# EUROPÄISCHE PATENTANMELDUNG

(11) **EP 3 839 052 A1**
(43) Veröffentlichungstag der Anmeldung: **23.06.2021**
(21) Anmeldenummer: 19218421.6
(22) Anmeldetag: 20.12.2019
(51) Int. Cl.: C12P 7/64, C12P 19/44

(54) **VERFAHREN ZUR ENZYMATISCHEN HERSTELLUNG VON ZUCKER-ESTERN UND/ODER ZUCKERALKOHOL-ESTERN**

(71) Anmelder: Evonik Operations GmbH, 45128 Essen (DE)
(72) Erfinder: VON HOF, Jan Marian, 44789 Bochum (DE); LIEBIG, Stefan Julian, 40477 Düsseldorf (DE); WENK, Hans Henning, 45470 Mühlheim an der Ruhr (DE); ECKSTEIN, Marrit Friederike, 45289 Essen (DE); KARACOCUK, Sunay, 44625 Herne (DE); YAVORSKY, Maxim, 58453 Witten (DE)
(74) Vertreter: Evonik Patent Association

(57) **Zusammenfassung**

Gegenstand der Erfindung ist ein Verfahren zur enzymatischen Herstellung von Zucker-Estern und/oder Zuckeralkohol-Estern sowie Mischungszusammensetzungen enthaltend Zucker-Ester und/oder Zuckeralkohol-Ester.

## Beschreibung

### Gebiet der Erfindung

Gegenstand der Erfindung ist ein Verfahren zur enzymatischen Herstellung von Zucker-Estern und/oder Zuckeralkohol-Estern sowie Mischungszusammensetzungen enthaltend Zucker-Ester und/oder Zuckeralkohol-Ester.

### Stand der Technik

Fettsäureester von Zuckern und Zuckeralkoholen haben tensidische Eigenschaften und sind aufgrund Ihrer natürlichen Rohstoffbasis und Nachhaltigkeit insbesondere für Anwendungen im Lebensmittelbereich und in der kosmetischen Industrie geeignet.

Die klassische Synthese von Fettsäureestern von Zuckern oder Zuckeralkoholen erfolgt durch Reaktion der Zucker oder Zuckeralkohole mit Fettsäurechloriden in Gegenwart von Pyridin (Surfactants, K. Kosswig in Ullmann's Encyclopedia of Industrial Chemistry, Online, Wiley-VCH, Weinheim, 2000, https://doi.org/10.1002/14356007.a25_747).
Ein Nachteil dieses im Stand der Technik beschriebenen Verfahrens ist jedoch, dass der Einsatz solcher Lösungsmittel für Anwendungen im Lebensmittel- oder Kosmetikbereich nicht akzeptabel ist und außerdem eine entsprechende Abtrennung der Lösungsmittel zusätzliche Verfahrensschritte wie Kristallisation, Filtration oder Destillation erfordert. Ein weiterer Nachteil dieses im Stand der Technik beschriebenen Verfahrens ist die quantitative Freisetzung von HCl bei der Verwendung von Fettsäurechloriden, da HCl zu Korrosion an den metallischen Oberflächen der Reaktoren führen kann.

Ein alternatives Verfahren des Standes der Technik, welches insbesondere in industriellem Maßstab genutzt wird, setzt die Zucker oder Zuckeralkohole mit den freien Fettsäuren oder den Fettsäurealkylestern in Abwesenheit eines Lösungsmittels bei Temperaturen von 200-250 °C in Gegenwart von basischen Katalysatoren wie beispielsweise Natriumhydroxid um (Römpp, Georg Thieme Verlag KG, 2019 zum Stichwort "Sorbitane" und Surfactants, K. Kosswig in Ullmann's Encyclopedia of Industrial Chemistry, Online, Wiley-VCH, Weinheim, 2000, https://doi.org/10.1002/14356007.a25_747). Ein Nachteil dieses im Stand der Technik beschriebenen Verfahrens ist jedoch, dass bei der Umsetzung zum Beispiel von Sorbitol unter den genannten Bedingungen, Dehydratisierung der Zucker oder Zuckeralkohole als Nebenreaktion auftritt. Diese Nebenreaktion tritt in Gegenwart von sauren Katalysatoren sogar schon ab ca. 140 °C auf. So wird beispielsweise Sorbitol zunächst zu Sorbitan (durch Verlust von einem Molekül Wasser) und weiter zu Isosorbid (durch Verlust eines weiteren Moleküls Wasser) dehydratisiert. Die eingesetzten Zucker oder Zuckeralkohole verlieren so an Hydrophilie und sind damit immer weniger als hydrophile Kopfgruppen für Tenside geeignet. Ein weiterer Nachteil dieses im Stand der Technik beschriebenen Verfahrens ist, dass die auftretenden Nebenreaktionen zu einer dunklen Färbung der erhaltenen Produkte führen, was gegebenenfalls eine weitere Behandlung, zum Beispiel mit Bleichmitteln wie Wasserstoffperoxid oder Aktivkohle erfordert, um die erhaltenen Produkte zum Beispiel in kosmetischen Formulierungen einsetzen zu können. Ein weiterer Nachteil dieses im Stand der Technik beschriebenen Verfahrens ist, dass die auftretenden Nebenreaktionen zu einem schlechten Geruch bei den erhaltenen Produkten führen, der eine unerwünschte Wechselwirkung mit den in kosmetischen Formulierungen eingesetzten Parfüms eingehen kann.

Die durch Enzyme katalysierte Herstellung von Fettsäureestern von Zuckern oder Zuckeralkoholen ist in verdünnten Lösungen in organischen Lösungsmitteln wie zum Beispiel 2- Methyl-2-butanol, Pyridin, Dimethylformamid oder 2-Pyrrolidon beschrieben (A. Ducret, A. Giroux, M. Trani, and R. Lortie, Characterization of enzymatically prepared biosurfactants, J. Am. Oil Chem. Soc. 1996, 73, 109-113, doi: 10.1007/BF02523456; M. Therisod, A. M. Klibanov, Facile enzymatic preparation of monoacylated sugars in pyridine, J. Am. Chem. Soc., 1986, 108 (18), pp 5638-5640; J. Chem. Soc., Perkin Trans. 1, 1989,0, 1057-1061,10.1039/P19890001057; A. E. M. Janssen, C. Klabbers, M. C. R. Franssen, K. van't Riet, Enzymatic synthesis of carbohydrate esters in 2-pyrrolidone, Enzyme and Microbial Technology, 1991, 13 (7), 565-572). Ein Nachteil dieser im Stand der Technik beschriebenen Verfahren ist jedoch, dass die Verwendung solcher Lösungsmittel für Anwendungen im Lebensmittel- oder Kosmetikbereich nicht akzeptabel ist und außerdem eine entsprechende Abtrennung der Lösungsmittel zusätzliche hohe Mengen an Energie verbrauchenden Verfahrensschritte wie Kristallisation, Filtration oder Destillation erfordern.

Eine enzymatische Synthese von Fettsäureestern von Zuckern oder Zuckeralkoholen unter Einsatz von verdünnten wässrigen Lösungen der Enzyme wird beschrieben in A. E. M. Janssen, A. G. Lefferts, K. van't Riet, Enzymatic synthesis of carbohydrate esters in aqueous media, Biotechnology Letters 1990, 12 (10), 711-716, DOI https://doi.org/10.1007/BF01024726; H. Seino, T. Uchibori, T. Nishitani, et al., Enzymatic synthesis of carbohydrate esters of fatty acid (I) esterification of sucrose, glucose, fructose and sorbitol, J. Am. Oil Chem. Soc. 1984, 61 1761-1765, https://doi.org/10.1007/BF02582144 und in WO9412651. Nachteile dieses im Stand der Technik beschriebenen Verfahrens sind die zur Isolation der Produkte aus den wässrigen Systemen zusätzlich erforderlichen, energieaufwändigen Prozessschritte wie Kristallisation, Filtration oder Destillation, bei gepufferten wässrigen System darüber hinaus das Entfernen der Salzfracht. Ein weiterer Nachteil ist hier, dass die Gegenwart von Wasser bei der Verwendung von Fettsäuren als Acyldonor das Verschieben der Gleichgewichtslage hin zu den Produkten erschwert. Ein weiterer Nachteil ist, dass Enzyme meist ein Performance-Maximum bei einer bestimmten Wasseraktivität haben.

T. Itoh, lonic Liquids as Tool to Improve Enzymatic Organic Synthesis, Chemical Reviews 2017, 117, 10567-10607 offenbart die durch Enzyme katalysierte Herstellung von Fettsäureestern von Zuckern oder Zuckeralkoholen in ionischen Flüssigkeiten als Lösungsmittel. Ein Nachteil dieser im Stand der Technik beschriebenen Verfahren ist jedoch, dass zur Abtrennung der ionischen Flüssigkeiten mindestens ein zusätzlicher, energieintensiver Prozessschritt wie z.B. Kristallisation, Filtration oder Destillation erforderlich ist, da die ionischen Flüssigkeiten für Folgeanwendungen im Lebensmittelbereich oder in der kosmetischen Industrie nicht im Produkt verbleiben können. Ein weiterer Nachteil dieser im Stand der Technik beschriebenen Verfahren ist, dass die ionischen Flüssigkeiten aus petrochemischen Rohstoffen hergestellt werden, und deren Verwendung daher für natürliche und nachhaltige Anwendungen im Lebensmittelbereich oder in der kosmetischen Industrie nicht erwünscht ist. Ein weiterer Nachteil der im Stand dieser Technik beschriebenen Verfahren ist die Verwendung von Fettsäurevinylestern als Acyldonor, da diese während der Reaktion toxikologisch bedenklichen Acetaldehyd freisetzen, was die Handhabung im industriellen Maßstab erschwert und außerdem für Anwendungen im Lebensmittelbereich oder in der kosmetischen Industrie unerwünscht ist. Außerdem werden die Fettsäurevinylester in Gegenwart toxikologisch bedenklicher Metallkatalysatoren wie z.B. Quecksilber, Cadmium, Palladium oder Silbersalzen aus petrochemischen Rohstoffen wie z.B. Acetylen oder Ethylen hergestellt (G. Roscher, Vinyl Esters in Ullmann's Encyclopedia of Industrial Chemistry, Online, Wiley-VCH, Weinheim, 2012, DOI: 10.1002/14356007.a27_419), was eine Verwendung dieser Rohstoffe für natürliche und nachhaltige Anwendungen im Lebensmittelbereich oder in der kosmetischen Industrie ausschließt.

Die durch Enzyme katalysierte Herstellung von Fettsäureestern von Zuckern oder Zuckeralkoholen ist ebenfalls in Gegenwart von Cholinchlorid (oder anderen Ammonium- oder Phosphonium-Salzen) zur Bildung tiefeutektischer Gemische beschrieben (S. Siebenhaller, C. Muhle-Goll, B. Luy, F. Kirschhöfer, G. Brenner-Weiss, E. Hiller, et al., Sustainable enzymatic synthesis of glycolipids in a deep eutectic solvent system, J. Mol. Catal. B Enzym. 2016, 133, 281-287, doi: 10.1016/j.molcatb.2017.01.015). Ein Nachteil dieses im Stand der Technik beschriebenen Verfahrens ist jedoch, dass Cholinchlorid oder andere Ammonium- oder Phosphonium-Salze durch ihren Salzcharakter die Anwendungsprofile von Fettsäureestern von Zuckern oder Zuckeralkoholen negativ beeinflussen können, sofern sie im Produkt verbleiben. Ein weiterer Nachteil dieses im Stand der Technik beschriebenen Verfahrens ist, dass es sich bei der industriell verfügbaren Qualität des Cholinchlorids um einen petrochemischen Rohstoff handelt, dessen Anwesenheit im Produkt daher für natürliche und nachhaltige Anwendungen im Lebensmittelbereich oder in der kosmetischen Industrie nicht erwünscht ist. Daher ist ein weiterer Nachteil dieses im Stand der Technik beschriebenen Verfahrens, dass zur Abtrennung des Cholinchlorids oder anderer Ammonium- oder Phosphonium-Salze mindestens ein zusätzlicher Prozessschritt wie z.B. Kristallisation, Filtration oder Destillation erforderlich ist.

Die durch Enzyme katalysierte Veresterung eines einzelnen Zuckers enthalten in Honig oder Agavensirup mit Fettsäurevinylestern als Acyldonor wird beschrieben in S. Siebenhaller, J. Gentes, A. Infantes, C. Muhle-Goll, F. Kirschhöfer, G. Brenner-Weiß, K. Ochsenreither, C. Syldatk, Lipase-Catalyzed Synthesis of Sugar Esters in Honey and Agave Syrup, Front. Chem. 2018, 6, Artikel 24, 1-9, doi: 10.3389/fchem.2018.00024). Ein Nachteil dieses im Stand der Technik beschriebenen Verfahrens ist die Verwendung von Fettsäurevinylestern als Acyldonor, da diese während der Reaktion toxikologisch bedenklichen Acetaldehyd freisetzen, was die Handhabung im industriellen Maßstab erschwert und außerdem für Anwendungen im Lebensmittelbereich oder in der kosmetischen Industrie unerwünscht ist. Außerdem werden die Fettsäurevinylester in Gegenwart toxikologisch bedenklicher Metallkatalysatoren wie z.B. Quecksilber, Cadmium, Palladium oder Silbersalzen aus petrochemischen Rohstoffen wie z.B. Acetylen oder Ethylen hergestellt (G. Roscher, Vinyl Esters in Ullmann's Encyclopedia of Industrial Chemistry, Online, Wiley-VCH, Weinheim, 2012, DOI: 10.1002/14356007.a27_419), was eine Verwendung dieser Rohstoffe für natürliche und nachhaltige Anwendungen im Lebensmittelbereich oder in der kosmetischen Industrie ausschließt. Ein weiterer Nachteil dieses im Stand der Technik beschriebenen Verfahrens ist die Verwendung von maximal nur 0,066 Äquivalenten des Acyldonors bezogen auf die Gesamtmenge an Zuckern und Zuckeralkoholen. Ein weiterer Nachteil dieses im Stand der Technik beschriebenen Verfahrens ist, dass der große Überschuss an den eingesetzten Zuckern und Zuckeralkoholen zur Folge hat, dass zur Isolation der Fettsäureester der Zucker und Zuckeralkohole mindestens ein weiterer zusätzlicher Prozessschritt wie z.B. Extraktion, Kristallisation, Filtration oder Destillation erforderlich ist. Ein weiterer Nachteil dieses im Stand der Technik beschriebenen Verfahrens ist, dass der große Überschuss an den eingesetzten Zuckern und Zuckeralkoholen im industriellen Maßstab unwirtschaftlich ist und darüber hinaus ein aufwändiges Rezyklieren der eingesetzten Zucker und Zuckeralkohole erforderlich macht. Ein weiterer Nachteil dieses im Stand der Technik beschriebenen Verfahrens ist, dass im Fall von Honig als Substrat lediglich Glucose-Ester und im Fall von Agave-Sirup als Substrat lediglich Fructose-Ester nachgewiesen werden, also jeweils nur eine der im Honig oder Agavensirup enthaltenen Zuckerkomponenten tatsächlich verestert wird.

Aufgabe der Erfindung war es, ein Verfahren zur Herstellung von Zucker-Estern und/oder Zuckeralkohol-Estern bereitzustellen, welches mindestens einen Nachteil der Verfahren des Standes der Technik zu überwinden vermag.

### Beschreibung der Erfindung

Überraschenderweise wurde gefunden, dass das im Folgenden beschriebene Verfahren die der Erfindung gestellt Aufgabe zu lösen vermag.

Gegenstand der vorliegenden Erfindung ist ein Verfahren zur enzymatischen Herstellung einer Mischungszusammensetzung umfassend mindestens zwei ausgewählt aus Zucker-Estern und/oder Zuckeralkohol-Estern umfassend den Verfahrensschritt
B) Umsetzen einer Mischung enthaltend mindestens zwei ausgewählt aus Zuckern und Zuckeralkoholen
mit mindestens einem Acylgruppendonor, bevorzugt Fettsäure-Acylgruppendonor, insbesondere ausgewählt aus Fettsäureestern und Fettsäuren, besonders bevorzugt Fettsäuren,
in Gegenwart einer Lipase.

Ein weiterer Gegenstand der Erfindung sind Mischungszusammensetzungen enthaltend bestimmte Zucker-Ester und/oder Zuckeralkohol-Ester.

Ein Vorteil der vorliegenden Erfindung ist es, dass das erfindungsgemäße Verfahren in Abwesenheit eines Lösungsmittels durchgeführt werden kann.
Noch ein Vorteil der vorliegenden Erfindung ist es, dass das erfindungsgemäße Verfahren unter Einsatz von natürlichen und nachhaltigen Synthesebausteinen durchgeführt werden kann.
Ein weiterer Vorteil der vorliegenden Erfindung ist es, dass die erfindungsgemäßen Zucker-Ester und/oder Zuckeralkohol-Ester in homogenen Reaktionsgemischen erhalten werden, und diese Eigenschaft schon bei niedrigen Veresterungsgraden erreicht werden kann.
Ein weiterer Vorteil der vorliegenden Erfindung ist es, dass die erfindungsgemäßen Zucker-Ester und/oder Zuckeralkohol-Ester hervorragende Farbeigenschaften aufweisen.
Ein weiterer Vorteil der vorliegenden Erfindung ist es, dass die erfindungsgemäßen Zucker-Ester und/oder Zuckeralkohol-Ester einen geringen Geruch aufweisen; insbesondre ist ein karamelltypischer Geruch kaum wahrnehmbar.
Ein Vorteil der vorliegenden Erfindung ist, dass Substrate in Mischung erfolgreich umgesetzt werden können, wohin gehend deren alleinige Umsetzung in Abwesenheit eines Lösungsmittels nicht erfolgreich ist.
Ein weiterer Vorteil der vorliegenden Erfindung ist, dass aus den eingesetzten Zuckern/Zuckeralkoholen keine unerwünschten Nebenprodukte unter Wasserabspaltung, wie z.B. Sorbitane aus Sorbitol gebildet werden.
Ein weiterer Vorteil der vorliegenden Erfindung ist, dass sich die erhaltenen Zucker-Ester und/oder Zuckeralkohol-Ester sehr leicht in Formulierungen, besonders in kosmetische Formulierungen einarbeiten lassen.
Ein weiterer Vorteil der vorliegenden Erfindung ist, dass sich mit den erhaltenen Zucker-Estern und/oder Zuckeralkohol-Estern milde Formulierungen herstellen lassen.
Ein weiterer Vorteil der vorliegenden Erfindung ist, dass sich mit den erhaltenen Zucker-Estern und/oder Zuckeralkohol-Estern Formulierungen mit besonders gutem Hautgefühl herstellen lassen. Ein weiterer Vorteil der vorliegenden Erfindung ist, dass sich mit den erhaltenen Zucker-Estern und/oder Zuckeralkohol-Estern nachhaltige Formulierungen ohne petrochemische Komponenten herstellen lassen.

Ein weiterer Vorteil der vorliegenden Erfindung ist, dass sich die erhaltenen Zucker-Ester und/oder Zuckeralkohol-Ester ohne die quantitative Freisetzung von HCl oder Acetaldehyd herstellen lassen. Ein weiterer Vorteil der vorliegenden Erfindung ist, dass die Umsetzung aufgrund der guten Durchmischbarkeit des Reaktionsansatzes in einer Blasensäule erfolgen kann, wodurch längere Katalysatorstandzeiten erreicht werden können.
Ein weiterer Vorteil der vorliegenden Erfindung ist, dass eine große Menge an Acyldonoren bezogen auf die gesamte Stoffmenge an Zuckern und/oder Zuckeralkoholen eingesetzt werden kann.
Ein weiterer Vorteil der vorliegenden Erfindung ist, dass die Ester der eingesetzten Zucker und Zuckeralkohole in einem homogenen Reaktionsgemisch erhalten werden, so dass keine zusätzlichen Prozessschritte wie z.B. Extraktion, Kristallisation, Filtration oder Destillation erforderlich sind.
Ein weiterer Vorteil der vorliegenden Erfindung ist, dass bei der Umsetzung mehr als nur eine der eingesetzten Zucker- und Zuckeralkoholkomponenten verestert wird.
Ein weiterer Vorteil der vorliegenden Erfindung ist, dass bei der Umsetzung bei niedrigeren Veresterungsgraden homogene Schmelzen erhalten werden.

Gegenstand der vorliegenden Erfindung ist ein Verfahren zur enzymatischen Herstellung einer Mischungszusammensetzung umfassend mindestens zwei ausgewählt aus Zucker-Estern und/oder Zuckeralkohol-Estern umfassend den Verfahrensschritt
B) Umsetzen einer Mischung enthaltend mindestens zwei ausgewählt aus Zuckern und Zuckeralkoholen
mit mindestens einem Acylgruppendonor, bevorzugt Fettsäure-Acylgruppendonor, insbesondere ausgewählt aus Fettsäureestern und Fettsäuren, besonders bevorzugt Fettsäuren,
in Gegenwart einer Lipase.

Unter dem Begriff "zwei ausgewählt aus Zucker-Estern und/oder Zuckeralkohol-Estern" im Zusammenhang mit der vorliegenden Erfindung ist zu verstehen, dass die zwei Ester sich hinsichtlich ihrer Zucker beziehungsweise hinsichtlich ihrer Zuckeralkohole unterscheiden. Es müssen daher Ester enthalten sein, die zwei unterschiedliche Reste bezüglich Zucker- und/oder Zuckeralkoholrest aufweisen.
Alle angegebenen Prozent (%) sind, wenn nicht anders angegeben, Massenprozent.

Erfindungsgemäß bevorzugt sind die Zucker und Zuckeralkohole aus der Gruppe von Zucker und Zuckeralkoholen enthaltend 4 bis 12 Kohlenstoffatome.
Erfindungsgemäß bevorzugt sind die Zucker und Zuckeralkohole ausgewählt aus der Gruppe Agarose, Allitol, Allulose, Altritol, Amylopectin, Amylose, Arabinitol, Arabinose, Cellobiose, Cellulose, Chitin, Cyclodextrine, Desoxyribose, Dextrane, Erythritol, Fructane, Fructose, Fucose, Galactitol, Galactose, Glucitol, Glucose, Glycogen, Hyaluronsäure, Iditol, Inulin, Isomalt, Isomaltulose, Isomelizitose, Lactitol, Lactose, Lactulose, Maltitol, Maltohexose, Maltopentose, Maltose, Maltotetrose, Maltotriose, Maltulose, Mannitol, Mannose, Melizitose, Pektine, Raffinose, Rhamnose, Ribitol, Ribose, Saccharose, Sorbitol, Sorbose, Stachyose, Stärke, Stärke-Hydrolysate, Threitol, Trehalulose, Umbelliferose, Xylitol und Xylose.
Erfindungsgemäß besonders bevorzugt sind die Zucker und Zuckeralkohole ausgewählt aus der Gruppe Allitol, Allulose, Altritol, Arabinitol, Arabinose, Cellobiose, Desoxyribose, Erythritol, Fructose, Fucose, Galactitol, Galactose, Glucitol, Glucose, Iditol, Isomalt, Isomaltulose, Lactitol, Lactose, Lactulose, Maltitol, Maltose, Maltulose, Mannitol, Mannose, Rhamnose, Ribitol, Ribose, Saccharose, Sorbitol, Sorbose, Threitol, Trehalulose, Xylitol und Xylose.
Erfindungsgemäß ganz besonders bevorzugt sind die Zucker und Zuckeralkohole ausgewählt aus Erythritol, Fructose, Glucose, Isomalt, Isomaltulose, Lactitol, Lactose, Maltitol, Maltose, Maltulose, Mannitol, Saccharose, Sorbitol, Sorbose, Xylitol und Xylose.
Erfindungsgemäß insbesondere bevorzugt sind die Zucker und Zuckeralkohole ausgewählt aus Erythritol, Fructose, Glucose, Sorbitol, Xylitol und Xylose.
Ein erfindungsgemäß bevorzugtes Verfahren ist dadurch gekennzeichnet, dass in Verfahrensschritt B) als Mischung enthaltend mindestens zwei ausgewählt aus Zuckern und Zuckeralkoholen Mischungen enthaltend
Glucose, Fructose und Maltose mit jeweils bezogen auf alle in der Mischung enthaltenen Zucker und Zuckeralkohole einem Glucosegehalt von 40 Gew.-% bis 50 Gew.-% und einem Fructosegehalt von 47 Gew.-% bis 57 Gew.-% sowie
Glucose, Fructose und Saccharose mit jeweils bezogen auf alle in der Mischung enthaltenen Zucker und Zuckeralkohole einem Glucosegehalt von 5 Gew.-% bis 24 Gew.-% und einem Fructosegehalt von 75 Gew.-% bis 94 Gew.-%
ausgenommen sind.

Es können sämtliche Acylgruppendonoren erfindungsgemäß eingesetzt werden. Solche sind beispielsweise Carbonsäureester oder Carbonsäuren selber sowie Mischungen davon. Erfindungsgemäß bevorzugt als Acylgruppendonor eingesetzte Carbonsäureester sind ausgewählt aus Estern auf Basis von Alkanolen und Polyolen mit bis zu 6 C-Atomen, besonders bevorzugt mit bis zu 3 C-Atomen, ganz besonders bevorzugt Glycerinester.
Insbesondere erfindungsgemäß bevorzugt als Acylgruppendonor eingesetzte Carbonsäureester sind ausgewählt aus Triglyceriden, insbesondere natürlichen Fetten und Ölen, besonders bevorzugt ausgewählt aus der Gruppe umfassend, bevorzugt bestehend aus, Kokosfett, Palmkernöl, Olivenöl, Palmöl, Arganöl, Rizinusöl, Leinöl, Babassuöl, Rapsöl, Algenöle, Sesamöl, Sojaöl, Avocadoöl, Jojobaöl, Diestelöl, Mandelöl, Baumwollsaatöl, Sheabutter, Sonnenblumenöl, Cupuaccubutter und Öle mit einem hohen Anteil an mehrfach ungesättigten Fettsäuren (PUFAS) eingesetzt. Ebenfalls bevorzugt eingesetzt werden können Sorbitanester, Monoglyceride und Diglyceride, insbesondere mit der im Folgenden beschriebenen Acylgruppen.

Erfindungsgemäß bevorzugt wird der Acylgruppendonor ausgewählt aus Fettsäure-Acylgruppendonoren, die insbesondere eine Acylgruppe ausgewählt aus der Gruppe der Acylgruppen von natürlichen Fettsäuren bereitstellen. Natürliche Fettsäuren lassen sich auf Basis natürlich vorkommender pflanzlicher oder tierischer Öle darstellen und weisen bevorzugt 6-30 Kohlenstoffatomen, insbesondere 8-22, Kohlenstoffatome auf. Natürliche Fettsäuren sind in der Regel unverzweigt und bestehen meist aus einer geraden Anzahl Kohlenstoffatomen. Eventuelle Doppelbindungen besitzen cis-Konfiguration. Beispiele sind: Capronsäure, Caprylsäure, Caprinsäure, Laurinsäure, Myristinsäure, Palmitinsäure, Palmitoleinsäure, Pelargonsäure (erhältlich aus der Ozonolyse von Ölsäure), Isostearinsäure, Stearinsäure, 12-Hydroxystearinsäure, Dihydroxystearinsäure, Undecylensäure (erhältlich aus der Pyrolyse von Rizinolsäure), Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure und Arachidonsäure.

Erfindungsgemäß bevorzugt als Acylgruppendonor werden Carbonsäuren, insbesondere Fettsäuren eingesetzt, wobei die konkret vorgenannten Fettsäuren besonders bevorzugt eingesetzt werden.

Es ist erfindungsgemäß bevorzugt, dass Vinyl-Ester als Acylgruppendonoren ausgeschlossen sind, weil diese während der Reaktion toxikologisch bedenklichen Acetaldehyd freisetzen, was die Handhabung im industriellen Maßstab erschwert und außerdem für Anwendungen im Lebensmittelbereich oder in der kosmetischen Industrie unerwünscht ist. Außerdem werden die Fettsäurevinylester in Gegenwart toxikologisch bedenklicher Metallkatalysatoren wie z.B. Quecksilber, Cadmium, Palladium oder Silbersalzen aus petrochemischen Rohstoffen wie z.B. Acetylen oder Ethylen hergestellt (G. Roscher, Vinyl Esters in Ullmann's Encyclopedia of Industrial Chemistry, Online, Wiley-VCH, Weinheim, 2012, DOI: 10.1002/14356007.a27_419), was eine Verwendung dieser Rohstoffe für natürliche und nachhaltige Anwendungen im Lebensmittelbereich oder in der kosmetischen Industrie ausschließt.

Insbesondere ist es erfindungsgemäß bevorzugt, dass die Zucker und Zuckeralkohole ausgewählt sind aus Erythritol, Fructose, Glucose, Sorbitol, Xylitol und Xylose und der Acylgruppendonor ausgewählt ist aus mindestens einer aus der Gruppe Capronsäure, Caprylsäure, Pelargonsäure, Caprinsäure, Undecylensäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure und Arachidonsäure.

Ein erfindungsgemäß bevorzugtes Verfahren ist dadurch gekennzeichnet, dass die in Verfahrensschritt B) eingesetzte Mischung enthaltend mindestens zwei ausgewählt aus Zuckern und Zuckeralkoholen Substanzen ausgewählt aus der Gruppe bestehend aus Cholin-, Ammonium- und Phosphonium-Salzen in einer Menge kleiner 2 Gew.-%, bevorzugt weniger als 1 Gew.-%, besonders bevorzugt weniger als 0,1 Gew.-%, insbesondere keine der Substanzen, aufweist, wobei sich die Gewichtsprozente auf alle Zucker und Zuckeralkohole in Verfahrensschritt B) in der Mischung enthaltend mindestens zwei ausgewählt aus Zuckern und Zuckeralkoholen beziehen.

Ein erfindungsgemäß bevorzugtes Verfahren ist dadurch gekennzeichnet, dass in Verfahrensschritt B) das molare Verhältnis von allen Zuckern und Zuckeralkoholen zu in allen Acylgruppendonoren enthaltenen Acylgruppen in einem Bereich von 1,00 zu 0,08 bis 1,00 zu 10,00 bevorzugt von 1,00 zu 0,50 bis 1,00 zu 7,00, besonders bevorzugt von 1,00 zu 1,25 bis 1,00 zu 2,25, alternativ besonders bevorzugt von 1,00 zu 2,00 bis 1,00 zu 4,50, liegt.

Ein erfindungsgemäß bevorzugtes Verfahren ist dadurch gekennzeichnet, dass in Verfahrensschritt B) das molare Verhältnis von allen primären Hydroxylgruppen in allen Zuckern und Zuckeralkoholen zu in allen Acylgruppendonoren enthaltenen Acylgruppen in einem Bereich von 1,00 zu 0,10 bis 1,00 zu 3,00, besonders bevorzugt von 1,00 zu 1,25 bis 1,00 zu 2,25, liegt.

Erfindungsgemäß bevorzugt eingesetzte Lipasen liegen auf einem festen Träger immobilisiert vor. Erfindungsgemäß bevorzugt eingesetzte Lipasen in Verfahrensschritt B) sind Lipasen ausgewählt aus der Gruppe umfassend die Lipase aus *Thermomyces lanuginosus (*accessionnumber *O59952),* die Lipasen A und B (accessionnumber P41365) aus *Candida antarctica* und, die Lipase aus *Mucor miehei (*accessionnumber *P19515),* die Lipase aus *Humicola sp.* (accessionnumber O59952), die Lipase aus *Rhizomucor javanicus (*accessionnumber S32492), die Lipase aus *Rhizopus oryzae* (accessionnumber P61872), die Lipasen aus *Candida rugosa* (accessionnumber P20261, P32946, P32947, P3294 und P32949), die Lipase aus *Rhizopus niveus (*accessionnumber *P61871),* die Lipase aus *Penicillium camemberti (*accessionnumber P25234), die Lipasen aus *Aspergillus niger* (ABG73613, ABG73614 und ABG37906) und die Lipase aus *Penicillium cyclopium (*accessionnumber P61869), sowie jeweils deren auf Aminosäureebene mindestens 60 %, vorzugsweise mindestens 80 % bevorzugt mindestens 90 %, besonders bevorzugt mindestens 95%, 98% bzw. 99 % homologen.
Die auf Aminosäureebene homologen Enzyme weisen bevorzugt verglichen zur Referenzsequenz mindestens 50 %, insbesondere mindestens 90 %, Enzymaktivität in im Zusammenhang mit der vorliegenden Erfindung definierten Propyllaurat Units auf.
Kommerzielle Beispiele und ebenfalls bevorzugt eingesetzte Carboxylester-Hydrolasen in erfindungsgemäßem Verfahren sind die Handelsprodukte Lipozyme TL IM, Novozym 435, Lipozyme IM 20, Lipase SP382, Lipase SP525, Lipase SP523, (alles Handelsprodukte der Firma Novozymes A/S, Bagsvaerd, Dänemark), Chirazyme L2, Chirazyme L5, Chirazyme L8, Chirazyme L9 (alles Handelprodukte der Firma Roche Molecular Biochemicals, Mannheim, Deutschland), CALB Immo Plus TM der Firma Purolite, sowie Lipase M "Amano", Lipase F-AP 15 "Amano", Lipase AY "Amano", Lipase N "Amano", Lipase R "Amano", Lipase A "Amano", Lipase D "Amano", Lipase G "Amano" (alles Handelsprodukte der Firma Amano, Japan),
Mit "Homologie auf Aminosäureebene" im Sinne der vorliegenden Erfindung wird die "Aminosäure-Identität" verstanden, welche mit Hilfe bekannter Verfahren bestimmt werden kann. Generell werden besondere Computerprogramme mit Algorithmen unter Berücksichtigung spezieller Erfordernisse verwendet. Bevorzugte Verfahren zur Bestimmung der Identität erzeugen zunächst die größte Übereinstimmung zwischen den zu vergleichenden Sequenzen. Computer-Programme zur Bestimmung der Identität umfassen, sind jedoch nicht beschränkt auf, das GCG-Programmpaket, einschließlich
- GAP (Deveroy, J. et al., Nucleic Acid Research 12 (1984), Seite 387, Genetics Computer Group University of Wisconsin, Medicine (WI), und
- BLASTP, BLASTN und FASTA (Altschul, S. et al., Journal of Molecular Biology 215 (1990), Seiten 403-410. Das BLAST-Programm kann erhalten werden vom National Center For Biotechnology Information (NCBI) und aus weiteren Quellen (BLAST Handbuch, Altschul S. et al., NCBI NLM NIH Bethesda ND 22894; Altschul S. et al., vorstehend).
Der Fachmann ist sich bewusst, dass verschiedene Computerprogramme für die Kalkulation der Ähnlichkeit bzw. Identität zwischen zwei Nukleotid- oder Aminosäure-Sequenzen zur Verfügung stehen. So kann die prozentuale Identität zwischen zwei Aminosäure-Sequenzen z.B. durch den Needleman und Wunsch (J. Mol. Biol. (48): 444-453 (1970)) Algorithmus bestimmt werden, der in das GAP Programm im GCG Software-Paket (erhältlich über http://www.gcg.com) integriert wurde, und zwar entweder unter Verwendung einer Blossom 62-Matrix oder einer PAM250-Matrix, einer gap weight von 16, 14, 12, 10, 8, 6, oder 4 und einer length weight von 1, 2, 3, 4, 5, oder 6. Der Fachmann wird anerkennen, dass die Verwendung unterschiedlicher Parameter zu leicht unterschiedlichen Ergebnissen führen wird, dass aber die prozentuale Identität zwischen zwei Aminosäure-Sequenzen insgesamt nicht signifikant unterschiedlich sein wird. Üblicherweise wird die Blossom 62-Matrix unter Anwendung der Voreinstellungen (*gap weight:* 12, *length weight:* 1) genutzt.
Eine Identität von 60 % gemäß dem vorstehenden Algorithmus bedeutet im Zusammenhang mit der vorliegenden Erfindung 60 % Homologie. Das gleiche gilt für höhere Identitäten.

Verfahrensschritt B) wird erfindungsgemäß bevorzugt bei Reaktionstemperaturen im Bereich zwischen 20 °C und 160 °C, bevorzugt 35 °C und 130°C, insbesondere zwischen 50 °C und 110 °C durchgeführt.

Verfahrensschritt B) wird erfindungsgemäß bevorzugt bei einem Druck von kleiner 1 bar, bevorzugt kleiner 0,5 bar und besonders bevorzugt kleiner 0,05 bar, durchgeführt.

Verfahrensschritt B) wird erfindungsgemäß alternativ bevorzugt in einem Blasensäulenreaktor durchgeführt, wobei der Reaktionsansatz mit mindestens einem Inertgas durchströmt wird; dieses ist bevorzugt ausgewählt aus der Gruppe umfassend, bevorzugt bestehend aus, Stickstoff und Argon. Es ist in diesem Zusammenhang erfindungsgemäß bevorzugt, wenn der Gasstrom 1 bis 60 kg/h, bevorzugt 5 bis 25 kg/h, noch mehr bevorzugt 10 bis 14 kg/h, beträgt.

Ein erfindungsgemäß bevorzugtes Verfahren ist dadurch gekennzeichnet, dass in Verfahrensschritt B) die Mischung enthaltend mindestens zwei ausgewählt aus Zuckern und Zuckeralkoholen sowie der Acylgruppendonor in Summe mindestens 10 Gew.-%, bevorzugt mindestens 86 Gew.-%, besonders bevorzugt mindestens 90 Gew.-%, des gesamten Reaktionsansatzes ausmachen.

Ein erfindungsgemäß bevorzugtes Verfahren ist dadurch gekennzeichnet, dass in Verfahrensschritt B) entstehende Nebenprodukte, beispielsweise im Falle, dass der eingesetzte Acylgruppendonor eine Säure ist, Wasser, im Falle, dass der eingesetzte Acylgruppendonor ein Ester ist, der korrespondierende Alkohol, entfernt werden.
Dies ist beispielsweise durch Destillation möglich.

Erfindungsgemäß bevorzugt umfasst das erfindungsgemäße Verfahren den Verfahrensschritt A) räumlich voneinander getrenntes Bereitstellen der mindestens zwei ausgewählt aus Zuckern und Zuckeralkoholen in fester oder wassergelöster Form und Vermengen derselben zu der in Verfahrensschritt B) eingesetzten Mischung enthaltend mindestens zwei ausgewählt aus Zuckern und Zuckeralkoholen. Hier kann es insbesondere bevorzugt sein, durch Wasserentfernung die Mischung enthaltend mindestens zwei ausgewählt aus Zuckern und Zuckeralkoholen sowie den Acylgruppendonor aufzukonzentrieren, um die vorgenannten in Summe mindestens 10 Gew.-%, bevorzugt mindestens 86 Gew.-%, besonders bevorzugt mindestens 90 Gew.-%, des gesamten Reaktionsansatzes zu erreichen.
In diesem Zusammenhang ist es insbesondere bevorzugt, dass Verfahrensschritt A) eine Reduktion des Wassergehaltes der in Verfahrensschritt B) eingesetzten Mischung enthaltend mindestens zwei ausgewählt aus Zuckern und Zuckeralkoholen auf weniger als 17 Gew. %, bevorzugt weniger als 14 Gew.-%, besonders bevorzugt weniger als 10 Gew.-%, umfasst, wobei sich die Gewichtsprozente auf die gesamte in Verfahrensschritt B) eingesetzte Mischung enthaltend mindestens zwei ausgewählt aus Zuckern und Zuckeralkoholen beziehen.

Erfindungsgemäß bevorzugt umfasst das erfindungsgemäße Verfahren den Verfahrensschritt C) Abtrennen der Lipase.

Ebenfalls erfindungsgemäß bevorzugt umfasst das erfindungsgemäße Verfahren den Verfahrensschritt D) Filtration der Mischungszusammensetzung umfassend mindestens zwei ausgewählt aus Zucker-Estern und/oder Zuckeralkohol-Estern durch einen Filter, insbesondere einen Beutelfilter, mit einer Feinheit von 0,1 µ bis 1250 µ, bevorzugt von 0,5 µ bis 100 µ. Verfahrensschritt D) wird erfindungsgemäß bevorzugt in einem Temperaturbereich von 20 °C bis 150 °C, insbesondere 40 °C bis 120 °C, durchgeführt.
Verfahrensschritt D) wird erfindungsgemäß bevorzugt in einem Druckbereich von 1 bar bis 25 bar, insbesondere von 1,5 bar bis 10 bar, durchgeführt.

Erfindungsgemäß bevorzugt umfasst das erfindungsgemäße Verfahren neben gegebenenfalls den Verfahrensschritten C) und D) keinen weiteren Reinigungsschritt.

Ein weiterer Gegenstand der vorliegenden Erfindung ist eine Mischungszusammensetzung umfassend mindestens zwei ausgewählt aus Zucker-Estern und Zuckeralkohol-Estern erhältlich nach dem erfindungsgemäßen Verfahren.

Noch ein Gegenstand der vorliegenden Erfindung ist eine Mischungszusammensetzung enthaltend Zucker-Ester und/oder Zuckeralkohol-Ester, dadurch gekennzeichnet, dass der Zucker- und/oder Zuckeralkoholrest des Zucker-Esters und/oder des Zuckeralkohol-Esters ausgewählt ist aus mindestens zwei Zucker- und/oder Zuckeralkoholresten ausgewählt aus der Gruppe der Reste von Allitol, Allulose, Altritol, Arabinitol, Arabinose, Cellobiose, Desoxyribose, Erythritol, Fructose, Fucose, Galactitol, Galactose, Glucitol, Glucose, Iditol, Iditol, Isomalt, Isomaltulose, Lactitol, Lactose, Lactulose, Maltitol, Maltose, Maltulose, Mannitol, Mannose, Rhamnose, Ribitol, Ribose, Saccharose, Sorbitol, Sorbose, Threitol, Trehalulose, Xylitol und Xylose,
bevorzugt von Erythritol, Fructose, Glucose, Isomalt, Isomaltulose, Lactitol, Lactose, Maltitol, Maltose, Maltulose, Mannitol, Saccharose, Sorbitol, Sorbose, Xylitol und Xylose, insbesondere bevorzugt von Erythritol, Fructose, Glucose, Sorbitol, Xylitol und Xylose, und
der Esterrest ausgewählt ist aus mindestens einer Acylgruppe der Gruppe der Säurereste der Fettsäuren,
bevorzugt der Capronsäure, Caprylsäure, Pelargonsäure, Caprinsäure, Undecylensäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure und Arachidonsäure.

Erfindungsgemäß bevorzugte Mischungszusammensetzungen enthalten den Zucker-Ester und/oder den Zuckeralkohol-Ester, in einer Menge von mindestens 50 Gew.-%, bevorzugt mindestens 80 Gew.-%, besonders bevorzugt mindestens 95 Gew.-%, wobei sich die Gewichtsprozente auf die Gesamtmischungszusammensetzung beziehen.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Methode zur Säurezahlbestimmung

Geeignete Methoden zur Bestimmung der Säurezahl sind insbesondere solche gemäß DGF C-V 2, DIN EN ISO 2114, Ph.Eur. 2.5.1, ISO 3682 und ASTM D 974.

### Methode zur Bestimmung der spezifischen Aktivität des eingesetzten Enzyms in PLU:

Zur Ermittlung der Enzymaktivität in PLU (Propyl Laurate Unit) werden 1-Propanol und Laurinsäure in äquimolarem Verhältnis bei 60°C homogen gemischt. Mit Enzymzugabe wird die Reaktion gestartet und die Reaktionszeit gestoppt. In Abständen werden Proben aus der Reaktionsmischung entnommen und der Gehalt an umgesetzter Laurinsäure mittels Titration mit Kaliumhydroxid-Lösung bestimmt. Die Enzymaktivität in PLU ergibt sich aus der Geschwindigkeit, in der 1 g des betrachteten Enzyms 1 µmol Propyllaurat pro min bei 60°C synthetisiert, vgl. hierzu auch US20070087418, insbesondere [0185].

### Methode zur Bestimmung der Farbzahlen

Ein Aliquot (ca. 10 g; so dass die Küvette ausreichend gefüllt ist) wurde bei 90 °C in einer 11 mm Rundküvette in einem Lico 690 Spektralcolorimeter vermessen, und die jeweils angegeben Farbzahlen protokolliert.

### Beispiel 1: Enzymatische Veresterung von Xylitol mit 2.00 Äq. Caprylsäure (nicht erfindungsgemäß)

Ein Gemisch aus Xylitol (60.0 g, 0.394 mol, 1.00 Äq.) und Caprylsäure (Säurezahl = 389 mg KOH/g, >98%,113.70 g, 0.788 mol, 2.00 Äq.) wurde unter Rühren und N₂-Durchleitung auf 80 °C erwärmt und nach 1 h wurde immobilisiertes Enzym *Candida antarctica* lipase B (5.21 g; Purolite D5619, entspricht 45110 PLU) zugegeben. Das Gemisch wurde bei 80 °C und 15 mbar für 24 h gerührt, und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, farblos und wies eine Säurezahl von 1.8 mg KOH/g auf.

### Beispiel 2: Enzymatische Veresterung von Fructose mit 2.00 Äq. Caprylsäure (nicht erfindungsgemäß)

Ein Gemisch aus Fructose (83.31 g, 0.462 mol, 1.00 Äq.) und Caprylsäure (Säurezahl = 389 mg KOH/g, >98%,133.36 g, 0.925 mol, 2.00 Äq.) wurde unter Rühren und N₂-Durchleitung auf 80 °C erwärmt, und nach 30 min wurde immobilisiertes Enzym *Candida antarctica* lipase B (6.50 g; Fermenta BIOCATALYST CALB_{TA} 10000 NLT 95%, entspricht 63788 PLU) zugegeben. Das Gemisch wurde bei 80 °C und 20 mbar für 24 h gerührt und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze inhomogen, rötlich gefärbt und wies eine Säurezahl von ca. 140.5 mg KOH/g auf (wegen der Inhomogenität war die Säurezahl nicht eindeutig bestimmbar).

### Beispiel 3: Physikalische Mischung von Beispiel 1 und Beispiel 2 (nicht erfindungsgemäß)

Ein Gemisch eines Esters erhalten wie beschrieben in Beispiel 1 (42.00 g) und eines Esters erhalten wie beschrieben in Beispiel 2 (18.00 g) wurde unter Rühren und N₂-Durchleitung für 1 h auf 80 °C erhitzt. Das erhaltene Produkt war in der Schmelze inhomogen, trüb, orange und wies eine Säurezahl von ca. 40 mg KOH/g auf (wegen der Inhomogenität war die Säurezahl nicht eindeutig bestimmbar).

### Beispiel 4: Enzymatische Veresterung eines Gemisches von Xylitol und Fructose (70:30) mit 2.00 Äq. Caprylsäure (erfindungsgemäß)

Ein Gemisch aus Xylitol (42.00 g, 0.276 mol), D-(-)-Fructose (18.00 g, 0.100 mol) und Caprylsäure (Säurezahl = 389 mg KOH/g, >98%,108.40 g, 0.752 mol, 2.00 Äq. bezogen auf die Gesamteinwaage an Xylitol und Fructose) wurde unter Rühren und N₂-Durchleitung auf 80 °C erwärmt, und nach 1 h wurde immobilisiertes Enzym *Candida antarctica* lipase B (5.05 g; Purolite D5619, entspricht 43724 PLU) zugegeben. Das Gemisch wurde bei 80 °C und 15 mbar für 27 h gerührt, und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, klar und gelblich und wies eine Säurezahl von 2.4 mg KOH/g auf.

### Beispiel 5: Enzymatische Veresterung von Xylitol mit 1.50 Äq. Caprylsäure (nicht erfindungsgemäß)

Ein Gemisch aus Xylitol (89.14 g, 0.586 mol, 1.00 Äq.) und Caprylsäure (Säurezahl = 389 mg KOH/g, >98%,126.7 g, 0.879 mol, 1.50 Äq.) wurde unter Rühren und N₂-Durchleitung auf 80 °C erhitzt, und nach 30 min wurde immobilisiertes Enzym *Candida antarctica* lipase B (6.47 g; Fermenta BIOCATALYST CALB_{TA} 10000 NLT 95%, entspricht 63493 PLU) zugegeben. Das Gemisch wurde bei 80 °C und 20 mbar für 24 h gerührt, und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, klar und farblos und wies eine Säurezahl von 1.3 mg KOH/g auf.

### Beispiel 6: Enzymatische Veresterung von Sorbitol mit 1.50 Äq. Caprylsäure (nicht erfindungsgemäß)

Ein Gemisch aus Sorbitol (98.09 g, 0.538 mol, 1.00 Äq.) und Caprylsäure (Säurezahl = 389 mg KOH/g, >98%, 116.47 g, 0.808 mol, 1.50 Äq.) wurde unter Rühren und N₂-Durchleitung auf 100 °C erhitzt und nach 30 min wurde immobilisiertes Enzym *Candida antarctica* lipase B (6.44 g; Purolite D5619, entspricht 55759 PLU) zugegeben. Anschließend wurde das Gemisch bei 90 °C und 50 mbar für 24 h gerührt und währenddessen kontinuierlich das entstehende Wasser abdestilliert. Das erhaltene Reaktionsgemisch war nach 24 h inhomogen, hellgelb und wies eine Säurezahl von ca. 10-11 mg KOH/g auf (wegen der Inhomogenität war die Säurezahl nicht eindeutig bestimmbar).

### Beispiel 7: Physikalische Mischung von Beispiel 5 und Beispiel 6

Ein Gemisch eines Esters erhalten wie beschrieben in Beispiel 5 (42.00 g) und eines Esters erhalten wie beschrieben in Beispiel 6 (18.00 g) wurde unter Rühren und N₂-Durchleitung für 1 h auf 80 °C erhitzt. Das erhaltene Produkt war in der Schmelze inhomogen, und wies eine Säurezahl von 3.7 mg KOH/g auf.

### Beispiel 8: Enzymatische Veresterung eines Gemisches von Xylitol und Sorbitol (70:30) mit 1.50 Äq. Caprylsäure (erfindungsgemäß)

Ein Gemisch aus Xylitol (64.15 g, 0.421 mol), Sorbitol (27.49 g, 0.151 mol) und Caprylsäure (Säurezahl = 389 mg KOH/g, >98%,123.81 g, 0.859 mol, 1.50 Äq. bezogen auf die Gesamteinwaage an Xylitol und Sorbitol) wurde unter Rühren und N₂-Durchleitung auf 80 °C erwärmt und nach 30 min wurde immobilisiertes Enzym *Candida antarctica* lipase B (6.02 g; Fermenta BIOCATALYST CALB_{TA} 10000 NLT 95%; entspricht 59077 PLU) zugegeben. Anschließend wurde das Gemisch bei 80 °C und 20 mbar für 24 h gerührt, und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, klar und farblos und wies eine Säurezahl von 1.6 mg KOH/g auf.

### Beispiel 9: Enzymatische Veresterung eines Gemisches von Xylitol und Sorbitol (66:34) mit 2.00 Äq. technischer Ölsäure (erfindungsgemäß)

Ein Gemisch aus Xylitol (11.72 g, 0.077 mol), Sorbitol (6.01 g, 0.033 mol) und Ölsäure (Säurezahl = 200 mg KOH/g, lodzahl = 92.3 g I₂/100 g, 61.7 g, 0.22 mol, 2.00 Äq. bezogen auf die Gesamteinwaage an Xylitol und Sorbitol) wurde unter Rühren und N₂-Durchleitung auf 90 °C erwärmt und nach 30 min wurde immobilisiertes Enzym *Candida antarctica* lipase B (2.3 g; Fermenta BIOCATALYST CALB_{TA} 10000 NLT 95%; entspricht 59077 PLU) zugegeben. Anschließend wurde das Gemisch bei 80 °C und 10 mbar für 24 h gerührt und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, leicht trüb und hellgelb und wies eine Säurezahl von 2.0 mg KOH/g auf.

### Beispiel 10: Enzymatische Veresterung eines Gemisches von Xylitol und Sorbitol (70:30) mit 2.00 Äq. technischer Ölsäure (erfindungsgemäß)

Ein Gemisch aus Xylitol (28.0 g, 0.184 mol), Sorbitol (12.0 g, 0.066 mol) und Ölsäure (Säurezahl = 200 mg KOH/g, lodzahl = 92.3 g I₂/100 g, 140.2 g, 0.50 mol, 2.00 Äq. bezogen auf die Gesamteinwaage an Xylitol und Sorbitol) wurde unter Rühren und N₂-Durchleitung auf 80 °C erwärmt und nach 1 h wurde immobilisiertes Enzym *Candida antarctica* lipase B (5.40 g; Purolite D5619, entspricht 46754 PLU) zugegeben. Anschließend wurde das Gemisch bei 80 °C und 25 mbar für 24 h gerührt und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, leicht trüb und hellgelb und wies eine Säurezahl von 1.9 mg KOH/g auf.

### Beispiel 11: Enzymatische Veresterung von Xylitol mit 2.00 Äq. Stearinsäure (nicht erfindungsgemäß)

Ein Gemisch aus Xylitol (40.00 g, 0.263 mol, 1.00 Äq.) und Stearinsäure (Säurezahl = 198 mg KOH/g, >92%, 148.18 g, 0.526 mol, 2.00 Äq.) wurde unter Rühren und N₂-Durchleitung auf 90 °C erhitzt und nach 1 h wurde immobilisiertes Enzym *Candida antarctica* lipase B (5.65 g; Purolite D5619, entspricht 48919 PLU) zugegeben. Anschließend wurde das Gemisch bei 90 °C und 15 mbar für 24 h gerührt, und währenddessen kontinuierlich das entstehende Wasser abdestilliert. Anschließend wurde das Gemisch bei 80 °C über einen Büchnertrichter mit Schwarzbandfilter filtriert, um das Enzym zu entfernen. Das erhaltene Produkt war in der Schmelze homogen, klar, hellgelb und wies eine Säurezahl von 1.3 mg KOH/g auf.

### Beispiel 12: Enzymatische Veresterung von Fructose mit 2.00 Äq. Stearinsäure (nicht erfindungsgemäß)

Ein Gemisch aus Fructose (42.00 g, 0.233 mol, 1.00 Äq.) und Stearinsäure (Säurezahl = 198 mg KOH/g, >92%, 132.42 g, 0.482 mol, 2.00 Äq.) wurde unter Rühren und N₂-Durchleitung auf 90 °C erhitzt und nach 1 h wurde immobilisiertes Enzym *Candida antarctica* lipase B (5.18 g; Fermenta BIOCATALYST CALB_{TA} 10000 NLT 95%, entspricht 50834 PLU) zugegeben. Anschließend wurde das Gemisch bei 90 °C und 15 mbar für 51 h gerührt, und währenddessen kontinuierlich das entstehende Wasser abdestilliert. Anschließend wurde das Gemisch bei 90 °C über einen Büchnertrichter mit Schwarzbandfilter filtriert, um das Enzym zu entfernen. Das erhaltene Produkt war in der Schmelze homogen, klar, orange-rot und wies eine Säurezahl von 14.7 mg KOH/g auf.

### Beispiel 13: Physikalische Mischung von Beispiel 11 und Beispiel 12

Ein Gemisch eines Esters erhalten wie beschrieben in Beispiel 11 (42.00 g) und eines Esters erhalten wie beschrieben in Beispiel 12 (18.00 g) wurde unter Rühren und N₂-Durchleitung für 1 h auf 80 °C erhitzt. Das erhaltene Produkt war in der Schmelze homogen, klar, orange und wies eine Säurezahl von 5.1 mg KOH/g auf.

### Beispiel 14: Enzymatische Veresterung eines Gemisches von Xylitol und Fructose (70:30) mit 2.00 Äq. Stearinsäure (erfindungsgemäß)

Ein Gemisch aus Xylitol (28.0 g, 0.184 mol) und Fructose (12.0 g, 0.067 mol) wurde unter Rühren auf 130 °C erhitzt und nach 2 h auf 90 °C abgekühlt. Anschließend wurde unter Rühren und N₂-Durchleitung Stearinsäure (Säurezahl = 198 mg KOH/g, ca. 95%, 142.14 g, 0.501 mol, 2.00 Äq) zugegeben. Nach Rühren für 30 min bei 90 °C wurde immobilisiertes Enzym *Candida antarctica* lipase B (Purolite D5619, 5.46 g, entspricht 47274 PLU) zugegeben. Anschließend wurde das Gemisch bei 90 °C und 10 mbar für 24 h gerührt, und währenddessen kontinuierlich das entstehende Wasser abdestilliert. Das Gemisch wurde anschließend bei 80 °C und 2 bar N₂-Druck über eine Filterpresse mit Seitz T-750 Tiefenfilter filtriert um das Enzym zu entfernen. Das erhaltene Produkt war in der Schmelze homogen, klar und wies eine Säurezahl von 3.2 mg KOH/g auf.

### Beispiel 15: Enzymatische Veresterung eines Gemisches von Xylitol, Sorbitol und Fructose (50:25:25) mit 1.91 Äq. Caprylsäure (erfindungsgemäß)

Ein Gemisch aus Xylitol (39.59 g, 0.260 mol), Sorbitol (19.80 g, 0.109 mol), Fructose (19.80 g, 0.110 mol) und Caprylsäure (Säurezahl = 389 mg KOH/g, >98%, 138.05 g, 0.957 mol, 1.91 Äq. bezogen auf die Gesamteinwaage an Xylitol, Sorbitol und Fructose) wurde unter Rühren und N₂-Durchleitung auf 100 °C erwärmt und für 1 h gerührt. Anschließend wurde das Gemisch auf 80 °C abgekühlt, immobilisiertes Enzym *Candida antarctica* lipase B (5.92 g; Purolite D5619, entspricht 51257 PLU) zugegeben, weiter bei 80 °C und 20 mbar für 24 h gerührt und währenddessen kontinuierlich das entstehende Wasser abdestilliert. Anschließend wurde das Gemisch bei 90 °C über einen Büchnertrichter mit Schwarzbandfilter filtriert, um das Enzym zu entfernen. Das erhaltene Produkt war in der Schmelze homogen, klar und gelb und wies eine Säurezahl von 3.1 mg KOH/g auf.

### Beispiel 16: Enzymatische Veresterung eines Gemisches von Xylitol, Sorbitol und Glucose (65:25:10) mit 1.91 Äq. Caprylsäure (erfindungsgemäß)

Ein Gemisch aus Xylitol (52.12 g, 0.342 mol), Sorbitol (20.05 g, 0.110 mol), Glucose (8.02 g, 0.045 mol) und Caprylsäure (Säurezahl = 389 mg KOH/g, >98%, 136.91 g, 0.949 mol, 1.91 Äq. bezogen auf die Gesamteinwaage an Xylitol, Sorbitol und Glucose) wurde unter Rühren und N₂-Durchleitung auf 100 °C erwärmt und für 1 h gerührt. Anschließend wurde das Gemisch auf 80 °C abgekühlt, immobilisiertes Enzym *Candida antarctica* lipase B (5.91 g; Purolite D5619, entspricht 51170 PLU) zugegeben, weiter bei 80 °C und 20 mbar für 24 h gerührt und währenddessen kontinuierlich das entstehende Wasser abdestilliert. Anschließend wurde das Gemisch bei 90 °C über einen Büchnertrichter mit Schwarzbandfilter filtriert, um das Enzym zu entfernen. Das erhaltene Produkt war in der Schmelze homogen, klar und hellgelb und wies eine Säurezahl von 10.0 mg KOH/g auf.

### Beispiel 17: Enzymatische Veresterung eines Gemisches von Xylitol und Sorbitol (70:30) mit 1.50 Äq. Laurinsäure (erfindungsgemäß)

Ein Gemisch aus Xylitol (51.7 g, 0.340 mol), Sorbitol (22.16 g, 0.122 mol) und Laurinsäure (Säurezahl = 280 mg KOH/g, >99%,138.60 g, 0.692 mol, 1.50 Äq. bezogen auf die Gesamteinwaage an Xylitol und Sorbitol) wurde unter Rühren und N₂-Durchleitung auf 100 °C erwärmt und nach 60 min wurde immobilisiertes Enzym *Candida antarctica* lipase B (6.02 g; Purolite D5619, entspricht 52122 PLU) zugegeben. Anschließend wurde das Gemisch bei 95 °C und 50 mbar für 24 h gerührt, und währenddessen kontinuierlich das entstehende Wasser abdestilliert. Anschließend wurde das Gemisch bei 90 °C über einen Büchnertrichter mit Schwarzbandfilter filtriert, um das Enzym zu entfernen. Das erhaltene Produkt war in der Schmelze homogen, leicht trüb und hellgelb bis fast farblos und wies eine Säurezahl von 0.8 mg KOH/g auf.

### Beispiel 18: Differenzierung der erfindungsgemäßen Beispiele gegen den Stand der Technik

Die folgenden Beispiele sollen den Gegenstand der Erfindung näher erläutern ohne ihn auf diese Beispiele einzuschränken. Diese Beispiele sollen zeigen, dass das erfindungsgemäße Verfahren Vorteile in Bezug auf den Stand der Technik hat. Als Repräsentanten für den Stand der Technik wurden dabei die nicht erfindungsgemäßen Beispiele gewählt.

### Technischer Effekt: Homogenität & Geruch

In Tabelle 1 wird das erfindungsgemäße Beispiel 4 mit den nicht erfindungsgemäßen Beispielen 1, 2 und 3 bezüglich Reaktionsverlauf, Homogenität und Geruch verglichen.

**Tabelle 1:**

| | Beispiel 1 | Beispiel 2 | Beispiel 3 | Beispiel 4 |
|---|---|---|---|---|
| | Nicht erfindungsgemäß | Nicht erfindungsgemäß | Nicht erfindungsgemäß | erfindungsgemäß |
| Zucker/Zuckeralkohol | Xylitol | Fructose | Xylitol/Fructose | Xylitol/Fructose (70:30) |
| Fettsäure | Caprylsäure | Caprylsäure | Caprylsäure | Caprylsäure |
| Stoffmengenverhältnis Zucker / Fettsäure | 1:2 | 1:2 | 1:2 | 1:2 |
| Aussehen in der Schmelze nach Filtration bzw. nach Abmischung | Homogen | Inhomogen / 2 Phasen | Inhomogen / 2 Phasen | homogen |
| Säurezahl [mg KOH/g] nach dem Beenden der Reaktion | SZ <1.3 nach 24 h | SZ 140.5 nach 24 h | SZ ca. 40 | SZ 2.2 nach 24 h |
| Geruch nach dem Beenden der Reaktion & nach dem Abkühlen auf Raumtemperatur | Geruch nach Fettsäure | Geruch nach Fettsäure | Geruch nach Fettsäure | Geruch nach Popcorn |

Wie aus Tabelle 1 zu entnehmen ist, ist das nicht erfindungsgemäße Beispiel 2 nach 24 h Reaktionszeit inhomogen, weist noch eine hohe Restsäurezahl von ca. 140.5 mg KOH/g und darüber hinaus noch einen ausgeprägten Geruch nach Fettsäure auf, was bei den kurzkettigen Fettsäuren wie Caprylsäure als unangenehm empfunden wird. Dagegen ist zwar das nicht erfindungsgemäße Beispiel 1 nach 24 h Reaktionszeit homogen und weist eine Restsäurezahl von lediglich <1.3 mg KOH/g auf, jedoch lässt sich auch bei diesem Bespiel ein ausgeprägter Geruch nach Fettsäure feststellen. Gleiches gilt für die physikalische Mischung von Beispiel 1 und Beispiel 2 (Beispiel 3). Nur bei dem erfindungsgemäßen Beispiel 4 wird nach 24 h Stunden Reaktionszeit eine niedrige Restsäurezahl (also ein hoher Umsatz der Fettsäure), ein homogenes Produkt und ein angenehmer Geruch (popcornartig) erzielt.

### Technischer Effekt: Homogen auch bei niedrigeren Veresterungsgraden

In Tabelle 2 wird das erfindungsgemäße Beispiel 8 mit den nicht erfindungsgemäßen Beispielen 5 und 6 bezüglich Reaktionsverlauf und Homogenität verglichen.

**Tabelle 2:**

| | Beispiel 5 | Beispiel 6 | Beispiel 7 | Beispiel 8 |
|---|---|---|---|---|
| | Nicht erfindungsgemäß | Nicht erfindungsgemäß | Nicht erfindungsgemäß | erfindungsgemäß |
| Zucker/Zuckeralkohol | Xylitol | Sorbitol | Xyiltol/Sorbitol | Xyiltol/Sorbitol (70:30) |
| Fettsäure | Caprylsäure | Caprylsäure | Caprylsäure | Caprylsäure |
| Stoffmengenverhältnis Zucker / Fettsäure | 1:1.5 | 1:1.5 | 1:1.5 | 1:1.5 |
| Aussehen in der Schmelze nach dem Beenden der Reaktion bzw. nach Filtration | Ca. 2.5% (v/v) einer zweiten Phase am Boden | Ca. 30% (v/v) einer zweiten Phase am Boden | Ca. 10% (v/v) einer zweiten Phase am Boden | Klar, homogen |
| Säurezahl [mg KOH/g] nach dem Beenden der Reaktion | 1.3 | ca. 10-11 | ca. 3.7 | 1.6 |

Wie aus Tabelle 2 zu entnehmen ist, zeigt das nicht erfindungsgemäße Beispiel 5 bei 80 °C in der Schmelze eine Phasenseparation in Form eines Bodensatzes. Dieses Phänomen ist bei dem nicht erfindungsgemäßen Beispiel 6 sogar noch ausgeprägter und tritt auch bei der physikalischen Mischung aus Beispiel 5 und Beispiel 6 (Beispiel 7) auf. Lediglich das erfindungsgemäße Beispiel 8 ist bei 80 °C in der Schmelze homogen und zeigt keine Phasenseparation, trotz des vergleichsweise niedrigen Veresterungsgrads von 1:1.5 (Stoffmengenverhältnis Zucker/ Fettsäure).

### Technischer Effekt: Farbe und Reaktivität (d.h. Umsetzungsgrad der Fettsäure)

In Tabelle 3 wird das erfindungsgemäße Beispiel 14 mit dem nicht erfindungsgemäßen Beispiel 13 bezüglich Reaktionsverlauf und Farbe verglichen.

**Tabelle 3:**

| | Beispiel 13 | Beispiel 14 |
|---|---|---|
| | Nicht erfindungsgemäß | erfindungsgemäß |
| Zucker/Zuckeralkohol | Physik. Mischung | Xylitol/Fructose |
| Fettsäure | | Stearin 1892 |
| Stoffmengenverhältnis Zucker / Fettsäure | Ca. 70:30 Mischverhältnis | 1:2 |
| Farbe & Aussehen nach der Filtration & nach dem Abkühlen (qualitativ) | orange | Farblos bis hellgelb |
| Farbe in der Schmelze (quantitativ) | Hazen: 668 | Hazen: 227 |
| | Lovibond 5 ¼" Y: 31.0 | Lovibond 5 ¼" Y: 7.3 |
| | Lovibond 5 ¼" R: 3.3 | Lovibond 5 ¼" R: 1.7 |
| | Iodfarbzahl: 3.5 | Iodfarbzahl: 2.9 |
| | Gardner: 3.8 | Gardner: 3.3 |
| Säurezahl [mg KOH/g] nach dem Beenden der Reaktion | SZ 5.1 nach Abmischung | SZ 2.1 nach 24 h |

Wie aus Tabelle 3 zu entnehmen ist, weist die physikalische Mischung (Beispiel 13) eine deutlich schlechtere Farbe auf, als ein erfindungsgemäßes Verfahrensprodukt (Beispiel 14).

### Formulierungsbeispiele

Die folgenden Beispiele sollen zeigen, dass die erfindungsgemäßen Zusammensetzungen in einer Vielzahl kosmetischer Formulierungen eingesetzt werden können.

**Rezepturen 1a und 1b: Aluminiumsalzhaltige AP/Deo Formulierungen**

| **Rezeptur** | **1a** | **1b** |
|---|---|---|
| Zusammensetzung aus Beispiel 14 | 3,2% | 3,2% |
| C18-C22 Hydroxyalkyl Hydroxypropyl Guar (ESAFLOR HM 22, Lamberti S.p.A.) | 0,2% | |
| Hydroxypropyl Guar (ESAFLOR HDR, Lamberti S.p.A.) | | 0,2% |
| Isoamyl Cocoate (TEGOSOFT® AC, Evonik Nutrition & Care GmbH) | 5,4% | 5,4% |
| Wasser | Ad 100% | Ad 100% |
| Aluminum Chlorohydrate (50% aq.; Locron LIC, Clariant AG) | 20,0% | 20,0% |
| Methylisothiazolinone, Methylparaben, Ethylparaben; Dipropylene Glycol (Microcare MEM, Thor) | 0,8% | 0,8% |

**Rezepturen 2a und 2b: Aluminiumfreie Deo-Formulierung ohne AP-Wirkstoffe**

| **Rezeptur** | **2a** | **2b** |
|---|---|---|
| Zusammensetzung aus Beispiel 14 | 3,2% | 3,2% |
| Hydroxypropyl Guar (ESAFLOR HDR, Lamberti S.p.A.) | 0,15% | |
| Hydroxypropyl Guar (ESAFLOR HDR, Lamberti S.p.A.) | | 0,15% |
| Polyglyceryl-3 Caprylate (TEGO® Cosmo P 813, Evonik Nutrition & Care GmbH) | 0,5% | 0,5% |
| Zinc Ricinoleate (TEGODEO® PY 88 G, Evonik Nutrition & Care GmbH) | 1,0% | 1,0% |
| Caprylic/Capric Triglyceride (TEGOSOFT® CT, Evonik Nutrition & Care GmbH) | 5,65% | 5,65% |
| Wasser | Ad 100% | Ad 100% |
| Glycerin | 3,0% | 3,0% |
| Benzyl Alcohol, Benzoic Acid, Sorbic Acid (Rokonsal BSB-N, Ashland Specialty Ingredients) | 1,0% | 1,0% |
| Zitronensäure (50% aq.) | q.s. | q.s. |

**Rezepturen 3a und 3b: O/W Deodorant-Emulsion enthaltend Kalialaun**

| **Rezeptur** | **3a** | **3b** |
|---|---|---|
| Zusammensetzung aus Beispiel 14 | 4,8% | 4,8% |
| C18-C22 Hydroxyalkyl Hydroxypropyl Guar (ESAFLOR HDR, Lamberti S.p.A.) | 0,25% | |
| Hydroxypropyl Guar (ESAFLOR HDR, Lamberti S.p.A.) | | 0,25% |
| Isopropyl Palmitate (TEGOSOFT® P, Evonik Nutrition & Care GmbH) | 5,0% | 5,0% |
| Wasser | Ad 100% | Ad 100% |
| Glycerin | 3,0% | 3,0% |
| Kalialaun | 5,0% | 5,0% |
| Methylisothiazolinone, Methylparaben, Ethylparaben; Dipropylene Glycol (Microcare MEM, Thor) | 0,8% | 0,8% |

**Rezeptur 4: AP/Deo Lotion**

| | |
|---|---|
| Zusammensetzung aus Beispiel 14 | 3,0% |
| Diethylhexyl Carbonate (TEGOSOFT® DEC, Evonik Nutrition & Care GmbH) | 3,0% |
| PPG-14 Butyl Ether (TEGOSOFT® PBE, Evonik Nutrition & Care GmbH) | 3,0% |
| Polyglyceryl-3 Caprylate (TEGO® Cosmo P813, Evonik Nutrition & Care GmbH) | 0,5% |
| Demineralized Water | ad 100% |
| Hydroxyethylcellulose (Natrosol 250 HHR, Ashland Specialty Chemicals) | 1,0% |
| Aluminum Chlorohydrate (50%ig) (Reach 501L, Reheis) | 15,0% |
| Methylisothiazolinone, Methylparaben, Ethylparaben; Dipropylene Glycol (Microcare MEM, Thor) | 0,8% |

**Rezepturen 5a und 5b: AP/Deo Cremes**

| **Rezeptur** | **5a** | **5b** |
|---|---|---|
| Zusammensetzung aus Beispiel 14 | 3,0% | 2,5% |
| Sodium Cetearyl Sulfate (Lanette E, BASF SE) | | 0,5% |
| Glyceryl Stearate | 1,0% | 1,0% |
| Stearyl Alcohol | 1,0% | 1,0% |
| PPG-15 Stearyl Ether | 5,0% | 5,0% |
| Isoamyl Cocoate (TEGOSOFT® AC, Evonik Nutrition & Care GmbH) | 5,0% | 5,0% |
| Diethylhexyl Carbonate (TEGOSOFT® DEC, Evonik Nutrition & Care GmbH) | 5,0% | 5,0% |
| *Persea Gratissima (Avocado) Oil* | 2,0% | 2,0% |
| Polyglyceryl-3 Caprylate (TEGO® Cosmo P813, Evonik Nutrition & Care GmbH) | 0,5% | 0,5% |
| Zinc Ricinoleate (TEGODEO® PY88 G, Evonik Nutrition & Care GmbH) | 1,0% | 1,0% |
| Demineralized Water | ad 100% | ad 100% |
| Hydroxyethylcellulose (Natrosol 250 HHR (Ashland Specialty Chemicals) | 1,0% | 1,0% |
| Aluminum Chlorohydrate (50% aq.; Locron LIC, Clariant AG) | 15,0% | 15,0% |
| Methylisothiazolinone, Methylparaben, Ethylparaben; Dipropylene Glycol (Microcare MEM, Thor) | 0,8% | 0,8% |

**Rezeptur 6: Sun Care Spray SPF 30**

| | |
|---|---|
| Zusammensetzung aus Beispiel 14 | 4,0% |
| Phenoxyethyl Caprylate (TEGOSOFT® XC, Evonik Nutrition & Care GmbH) | 3,2% |
| Isopropyl Palmitate (TEGOSOFT® P, Evonik Nutrition & Care GmbH) | 2,0% |
| Bis-Ethylhexyloxyphenol Methoxyphenyl Triazine (Tinosorb S, BASF SE) | 3,0% |
| Butyl Methoxydibenzoylmethane | 2,0% |
| EHC | 2,0% |
| Ethylhexylsalicylat | 4,0% |
| Octocrylene | 4,0% |
| Glycerin | 3,0% |
| Wasser | ad 100% |
| Carbomersuspension 1 (Acrylates/C10-30 Alkyl Acrylate Crosspolymer, TEGO® Carbomer 341ER, Evonik Nutrition & Care GmbH, 20%ig in Phenoxyethyl Caprylate) | 1,0% |
| Tris(hydroxymethyl)-aminomethan (30% aq.) | 0,6% |
| UV-Filterlösung (20 % Phenylbenzimidazole Sulfonic Acid, 8,8% Tris (hydroxymethyl)-aminomethan, demineralisiertes Wasser ad 100%) | 10,0% |
| Methylisothiazolinone, Methylparaben, Ethylparaben; Dipropylene Glycol (Microcare MEM, Thor) | 0,8% |

**Rezepturen 7a und 7b: Sonnenschutzspray**

| **Rezeptur** | **7a** | **7b** |
|---|---|---|
| Zusammensetzung aus Beispiel 14 | 3,0% | 2,5% |
| Glyceryl Stearate Citrate (AXOL® C 62, Evonik Nutrition & Care GmbH) | | 0,5% |
| Glyceryl Stearate | 0,5% | 0,5% |
| Stearyl Alcohol | 0,5% | 0,5% |
| Bis-Ethylhexyloxyphenol Methoxyphenyl Triazine (Tinosorb S, BASF SE) | 3,0% | 3,0% |
| Butyl Methoxydibenzoylmethane | 2,0% | 2,0% |
| Ethylhexyl Methoxycinnamate | 2,0% | 2,0% |
| Ethylhexylsalicylat | 4,0% | 4,0% |
| Octocrylene | 4,0% | 4,0% |
| Isopropyl Palmitate | 2,0% | 2,0% |
| Phenoxyethyl Caprylate (TEGOSOFT® XC, Evonik Nutrition & Care GmbH) | 3,2% | 3,2% |
| Glycerin | 3,0% | 3,0% |
| Demineralized Water | ad 100% | ad 100% |
| Carbomersuspension (Acrylates/C10-30 Alkyl Acrylate Crosspolymer, TEGO® Carbomer 341ER, 20%ig in Phenoxyethyl Caprylate) | 1,0% | 1,0% |
| Tromethamin (30%ig) | 0,9% | 0,9% |
| UV-Filterlösung (20 % Phenylbenzimidazole Sulfonic Acid, 8,8% Tris (hydroxymethyl)-aminomethan, Demineralized Water ad 100%) | 10,0% | 10,0% |
| Methylisothiazolinone, Methylparaben, Ethylparaben; Dipropylene Glycol (Microcare MEM, Thor) | 0,8% | 0,8% |

**Rezepturen 8a und 8b: Sonnenschutzlotion SPF 30**

| **Rezeptur** | **8a** | **8b** |
|---|---|---|
| Zusammensetzung aus Beispiel 14 | 3,00% | 2,00% |
| Cetearyl Glucoside (TEGO® Care CG 90, Evonik Nutrition & Care GmbH) | | 0,50% |
| Phenoxyethyl Caprylate (TEGOSOFT® XC, Evonik Nutrition & Care GmbH) | 8,00% | 8,00% |
| Diethylamino Hydroxybenzoyl Hexyl Benzoate (Uvinul A Plus,.BASF SE) | 6,00% | 6,00% |
| Ethylhexyl Methoxycinnamate | 8,00% | 8,00% |
| Stearyl Alcohol | 1,00% | 1,00% |
| Glyceryl Stearate | 1,00% | 1,00% |
| Tocopheryl Acetate | 0,50% | 0,50% |
| Glycerin | 2,00% | 2,00% |
| Demineralized Water | ad 100% | ad 100% |
| Tromethamin | 0,90% | 0,90% |
| Phenylbenzimidazol Sulfonic Acid | 2,00% | 2,00% |
| Acrylates / C10-30 Alkyl Acrylate Crosspolymer (TEGO® Carbomer 341 ER, Evonik Nutrition & Care GmbH) | 0,30% | 0,30% |
| Sodium Hydroxide (10 % aq.) | q.s. | q.s. |
| Methylisothiazolinone, Methylparaben, Ethylparaben; Dipropylene Glycol (Microcare MEM, Thor) | 0,80% | 0,80% |

**Rezepturen 9a und 9b: Sonnenschutzlotion SPF 30, hoher UVA-Schutz**

| **Rezeptur** | **9a** | **9b** |
|---|---|---|
| Zusammensetzung aus Beispiel 14 | 3,00% | 2,50% |
| Sodium Cetearyl Sulfate (Lanette E, BASF SE) | | 0,50% |
| Phenoxyethyl Caprylate (TEGOSOFT® XC, Evonik Nutrition & Care GmbH) | 7,30% | 7,30% |
| Butyl Methoxydibenzoylmethane | 5,00% | 5,00% |
| Diethylhexyl Butamido Triazone (UVAsorb HEB, 3V Sigma) | 1,00% | 1,00% |
| Ethylhexyl Salicylate | 1,50% | 1,50% |
| Octocrylene | 3,50% | 3,50% |
| Titanium Dioxide; Diethylhexyl Carbonate; Polyglyceryl-6 Polyhydroxystearate (TEGO® Sun TDEC 45, Evonik Nutrition & Care GmbH) | 2,20% | 2,20% |
| Stearyl Alcohol | 1,00% | 1,00% |
| Nylon-10/10 (TEGOLON® ECO 10-10, Evonik Nutrition & Care GmbH) | 0,50% | 0,50% |
| Glyceryl Stearate | 1,00% | 1,00% |
| Tocopheryl Acetate | 0,50% | 0,50% |
| Glycerin | 2,00% | 2,00% |
| Demineralized Water | ad 100% | ad 100% |
| Citric Acid (50% aq.) | 0,10% | 0,10% |
| Bis-Ethylhexyloxyphenol Methoxyphenyl Triazine (Tinosorb S, BASF SE) | 20,00% | 20,00% |
| Acrylates / C10-30 Alkyl Acrylate Crosspolymer (TEGO® Carbomer 341 ER, Evonik Nutrition & Care GmbH) | 0,30% | 0,30% |
| Sodium Hydroxide (10% a.q.) | 0,90% | 0,90% |
| Methylisothiazolinone, Methylparaben, Ethylparaben; Dipropylene Glycol (Microcare MEM, Thor) | 0,80% | 0,80% |

**Rezepturen 10a und 10b: Sonnenschutzlotion SPF 30**

| **Rezeptur** | **10a** | **10b** |
|---|---|---|
| Zusammensetzung aus Beispiel 14 | 3,00% | 2,00% |
| Methylglucose Sesquistearate (TEGO® Care PS, Evonik Nutrition & Care GmbH) | | 1,00% |
| Phenoxyethyl Caprylate (TEGOSOFT® XC, Evonik Nutrition & Care GmbH) | 1,50% | 1,50% |
| Octocrylene | 10,00% | 10,00% |
| Butyl Methoxydibenzoylmethane | 3,50% | 3,50% |
| Titanium Dioxide; Diethylhexyl Carbonate; Polyglyceryl-6 Polyhydroxystearate (TEGO® Sun TDEC 45, Evonik Nutrition & Care GmbH) | 14,50% | 14,50% |
| Stearyl Alcohol | 0,20% | 0,20% |
| Glyceryl Stearate | 0,20% | 0,20% |
| Tocopheryl Acetate | 0,50% | 0,50% |
| Glycerin | 2,00% | 2,00% |
| Demineralized Water | ad 100% | ad 100% |
| Acrylates / C10-30 Alkyl Acrylate Crosspolymer (TEGO® Carbomer 341 ER, Evonik Nutrition & Care GmbH) | 0,20% | 0,20% |
| Sodium Hydroxide (10% aq.) | 0,60% | 0,60% |
| Methylisothiazolinone, Methylparaben, Ethylparaben; Dipropylene Glycol (Microcare MEM, Thor) | 0,80% | 0,80% |

**Rezepturen 11a und 11b: Sonnenschutzlotion SPF 50, hoher UVA-Schutz**

| **Rezeptur** | 11a | 111b |
|---|---|---|
| Zusammensetzung aus Beispiel 14 | 3,00% | 2,50% |
| Polyglyceryl-3 Methylglucose Distearate (TEGO® Care 450, Evonik Nutrition & Care GmbH) | | 0,50% |
| Phenoxyethyl Caprylate (TEGOSOFT® XC, Evonik Nutrition & Care GmbH) | 2,00% | 2,00% |
| Nylon-10/10 (TEGOLON® ECO 10-10, Evonik Nutrition & Care GmbH) | 0,50% | 0,50% |
| Butyl Methoxydibenzoylmethane | 5,00% | 5,00% |
| Diethylhexyl Butamido Triazone (UVAsorb HEB, 3V Sigma) | 1,00% | 1,00% |
| Bis-Ethylhexyloxyphenol Methoxyphenyl Triazine (Tinosorb S, BASF SE) | 3,00% | 3,00% |
| Ethylhexyl Salicylate | 1,00% | 1,00% |
| Octocrylene | 8,00% | 8,00% |
| Stearyl Alcohol | 0,45% | 0,45% |
| Glyceryl Stearate | 0,45% | 0,45% |
| Xanthan Gum (Keltrol CG-SFT, CP Kelco) | 0,30% | 0,30% |
| Tocopheryl Acetate | 0,50% | 0,50% |
| Hydroxyethylcellulose (Natrosol 250 HHR (Ashland Specialty Chemicals) | 0,50% | 0,50% |
| Demineralized Water | ad 100% | ad 100% |
| Sodium Hydroxide (10% aq., pH-Wert-Einstellung auf 7,5) | q.s. | q.s. |
| Disodium Phenyl Dibenzimidazole Tetrasulfonate (Neoheliopan AP, Symrise) | 5,00% | 5,00% |
| Methylene Bis-Benzotriazolyl Tetramethylbutylphenol (Tinosorb M, BASF SE) | 8,00% | 8,00% |
| Methylisothiazolinone, Methylparaben, Ethylparaben; Dipropylene Glycol (Microcare MEM, Thor) | 0,80% | 0,80% |

**Rezepturen 12a und 12b: Sonnenschutzlotion SPF 50**

| **Rezeptur** | **12a** | **12b** |
|---|---|---|
| Zusammensetzung aus Beispiel 14 | 3,50% | 3,00% |
| Glyceryl Stearate Citrate (AXOL® C 62, Evonik Nutrition & Care GmbH) | | 1,00% |
| Phenoxyethyl Caprylate (TEGOSOFT® XC, Evonik Nutrition & Care GmbH) | 2,50% | 2,50% |
| Diethylamino Hydroxybenzoyl Hexyl Benzoate | 10,00% | 10,00% |
| Ethylhexyl Methoxycinnamate | 10,00% | 10,00% |
| Stearyl Alcohol | 1,00% | 1,00% |
| Glyceryl Stearate | 1,00% | 1,00% |
| Xanthan Gum (Keltrol CG-SFT, CP Kelco) | 0,50% | 0,50% |
| Tocopheryl Acetate | 0,50% | 0,50% |
| Glycerin | 2,00% | 2,00% |
| Demineralized Water | ad 100% | ad 100% |
| Phenylbenzimidazol Sulfonic Acid | 4,00% | 4,00% |
| Tromethamin | 1,80% | 1,80% |
| Methylisothiazolinone, Methylparaben, Ethylparaben; Dipropylene Glycol (Microcare MEM, Thor) | 0,80% | 0,80% |

**Rezepturen 13a und 13b: Sonnenschutzlotion SPF 50+**

| **Rezeptur** | 13a | 13b |
|---|---|---|
| Zusammensetzung aus Beispiel 14 | 3,00% | 2,5% |
| Potassium Cetyl Phosphate | | 1,0% |
| Phenoxyethyl Caprylate (TEGOSOFT® XC, Evonik Nutrition & Care GmbH) | 3,00% | 3,00% |
| Butyl Methoxydibenzoylmethane | 5,00% | 5,00% |
| Octocrylene | 4,90% | 4,90% |
| Ethylhexyl Methoxycinnamate | 0,10% | 0,10% |
| Bis-Ethylhexyloxyphenol Methoxyphenyl Triazine (Tinosorb S, BASF SE) | 4,70% | 4,70% |
| Diethylhexyl Butamido Triazone (UVAsorb HEB, 3V Sigma) | 3,70% | 3,70% |
| Titanium Dioxide; Diethylhexyl Carbonate; Polyglyceryl-6 Polyhydroxystearate (TEGO® Sun TDEC 45, Evonik Nutrition & Care GmbH) | 11,00% | 11,00% |
| Stearyl Alcohol | 0,50% | 0,50% |
| Glyceryl Stearate | 0,50% | 0,50% |
| Xanthan Gum (Keltrol CG-SFT, CP Kelco) | 0,20% | 0,20% |
| Glycerin | 2,00% | 2,00% |
| Demineralized Water | ad 100% | ad 100% |
| Methylisothiazolinone, Methylparaben, Ethylparaben; Dipropylene Glycol (Microcare MEM, Thor) | 0,80% | 0,80% |

**Rezeptur 14: Körperlotion**

| | |
|---|---|
| Zusammensetzung aus Beispiel 14 | 4,0% |
| Isoamyl Cocoate (TEGOSOFT® AC, Evonik Nutrition & Care GmbH) | 2,5% |
| Caprylic/Capric Triglyceride (TEGOSOFT® CT, Evonik Nutrition & Care GmbH) | 3,5% |
| Wasser | ad 100% |
| Creatine (TEGO® Cosmo C 100, Evonik Nutrition & Care GmbH) | 0,5% |
| Carbomersuspension 2 (Carbomer, TEGO® Carbomer 141, Evonik Nutrition & Care GmbH, 20%ig in Ethylhexyl Stearate) | 1,0% |
| Natriumhydroxid (10% aq.) | 0,6% |
| Phenoxyethanol, Ethylhexylglycerin (Euxyl PE 9010, Schülke & Mayr GmbH) | 0,7% |

**Rezeptur 15: Natürliche Pflegecreme**

| | |
|---|---|
| Zusammensetzung aus Beispiel 14 | 6,0% |
| Caprylic/Capric Triglyceride (TEGOSOFT® CT, Evonik Nutrition & Care GmbH) | 8,0% |
| Isopropyl Palmitate (TEGOSOFT® P, Evonik Nutrition & Care GmbH) | 11,0% |
| Prunus Amygdalus Dulcis (Sweet Almond) Oil | 10,0% |
| Wasser | ad 100% |
| Glycerin | 3,0% |
| Natriumhydroxid (10% aq.) | 0,2% |
| Benzyl Alcohol, Benzoic Acid, Sorbic Acid (Rokonsal BSB-N, Ashland Specialty Ingredients) | 0,8% |

**Rezeptur 16: Anti-Aging Creme**

| | |
|---|---|
| Zusammensetzung aus Beispiel 14 | 6,0% |
| Caprylic/Capric Triglyceride (TEGOSOFT® CT, Evonik Nutrition & Care GmbH) | 9,5% |
| C12-15 Alkyl Benzoate (TEGOSOFT® TN, Evonik Nutrition & Care GmbH) | 9,5% |
| Wasser | ad 100% |
| Glycerin | 3,0% |
| Tetrapeptide-21; Glycerin; Butylene Glycol; Aqua (TEGO® PEP 4-17, Evonik Industries) | 4,0% |
| Sodium Hyaluronate (HyaCare®, Evonik Nutrition & Care GmbH) | 0,1% |
| Hydrolyzed Hyaluronic Acid (HyaCare® 50, Evonik Nutrition & Care GmbH) | 0,1% |
| Aqua; Ethylhexyl Stearate; Sodium Hyaluronate Crosspolymer; Polyglyceryl-4 Diisostearate/Polyhydroxystearate/Sebacate; Sodium Isostearate (HyaCare® Filler CL, Evonik Nutrition & Care GmbH) | 5,0% |
| Methylisothiazolinone, Methylparaben, Ethylparaben; Dipropylene Glycol (Microcare MEM, Thor) | 0,8% |

**Rezeptur 17: ONV Foundation**

| | |
|---|---|
| Zusammensetzung aus Beispiel 14 | 6,0% |
| Myristyl Myristate (TEGOSOFT® MM, Evonik Nutrition & Care GmbH) | 2,0% |
| Isopropyl Myristate (TEGOSOFT® M, Evonik Nutrition & Care GmbH) | 6,0% |
| Decyl Cocoate (TEGOSOFT® DC, Evonik Nutrition & Care GmbH) | 6,0% |
| Cetyl Ricinoleate (TEGOSOFT® CR, Evonik Nutrition & Care GmbH) | 1,0% |
| Wasser | ad 100% |
| Glycerin | 1,0% |
| Titanium Dioxide (Hombitan AC 360, Sachtleben) | 8,0% |
| Iron Oxides (Sicovit Gelb 10 E 172, Rockwood Pigments) | 0,9% |
| Iron Oxides (Sicovit Rot 30 E 172, Rockwood Pigments) | 0,2% |
| Iron Oxides (Sicovit Braun 70 E 172, Rockwood Pigments) | 0,4% |
| Iron Oxides (Sicovit Schwarz 80 E 172, Rockwood Pigments) | 0,1% |
| Cellulose (TEGO® Feel Green, Evonik Nutrition & Care GmbH) | 2,0% |
| Natriumhydroxid (10% aq.) | 0,2% |
| Benzyl Alcohol, Benzoic Acid, Sorbic Acid (Rokonsal BSB-N, Ashland Specialty Ingredients) | 1,0% |

**Rezepturen 18a, 18b und 18c: Lotionen mit kosmetischen Wirkstoffen**

| **Rezeptur** | **18a** | **18b** | **18c** |
|---|---|---|---|
| Zusammensetzung aus Beispiel 14 | 3,5% | 3,5% | 3,0% |
| Stearic Acid | | | 0,5% |
| Glyceryl Stearate | 0,5% | 0,6% | 0,6% |
| Cetearyl Alcohol | 0,5% | 0,6% | 0,6% |
| Caprylic/Capric Triglyceride | 8,5% | 8,5% | 8,5% |
| Ethylhexyl Palmitate (TEGOSOFT® OP, Evonik Nutrition & Care GmbH) | 8,5% | 8,5% | 8,5% |
| Demineralized Water | ad 100% | ad 100% | ad 100% |
| Xanthan Gum (Keltrol CG-SFT, CP Kelco) | 0,8% | 0,8% | 0,8% |
| Terminalia Arjuna Bark Extract; Pentylene Glycol (proposed; TEGO® Arjuna S, Evonik Nutrition & Care GmbH) | | 2,0% | 2,0% |
| Betaine; Urea; Potassium Lactate; Sodium Polyglutamate (proposed); Hydrolyzed Sclerotium Gum (TEGO® Smooth; Evonik Nutrition & Care GmbH) | 5,0% | | |
| Phenoxyethanol, Methylparaben, Ethylparaben, Propylparaben (Phenonip XB (Clariant International Ltd.) | 1,0% | | |
| Methylisothiazolinone, Methylparaben, Ethylparaben; Dipropylene Glycol (Microcare MEM, Thor) | | 0,8% | 0,8% |

**Rezepturen 19a und 19b: Lotion mit geringem Ölphasengehalt**

| **Rezeptur** | **19a** | **19b** |
|---|---|---|
| Zusammensetzung aus Beispiel 14 | 3,0% | 2,0% |
| Polyglyceryl-3 Dicitrate/Stearate (TEGO® Care PSC 3 (Evonik Nutrition & Care GmbH) | | 1,0% |
| Cetearyl Alcohol | 0,5% | 0,5% |
| Caprylic/Capric Triglyceride | 6,5% | 6,5% |
| Demineralized Water | ad 100% | ad 100% |
| Xanthan Gum (Keltrol CG-SFT, CP Kelco) | 0,5% | 0,5% |
| Methylisothiazolinone, Methylparaben, Ethylparaben; Dipropylene Glycol (Microcare MEM, Thor) | 0,8% | 0,8% |

**Rezepturen 20a, 20b und 20c: O/W Seren 1**

| **Rezeptur** | **20a** | **20b** | **20c** |
|---|---|---|---|
| Zusammensetzung aus Beispiel 14 | 2,5% | 2,5% | 4,0% |
| Sodium Stearoyl Glutamate (Eumulgin SG, BASF SE) | | 1,0% | |
| Glyceryl Stearate | | | 0,75% |
| Stearyl Alcohol | | | 0,75% |
| Caprylic/Capric Triglyceride | 2,0% | 2,0% | 2,0% |
| Oleyl Erucate (TEGOSOFT® OER, Evonik Nutrition & Care GmbH) | 2,0% | 2,0% | 2,0% |
| Isoamyl Cocoate (TEGOSOFT® AC, Evonik Nutrition & Care GmbH) | | | 3,0% |
| Persea Gratissima (Avocado) Oil | 1,0% | 1,0% | 1,0% |
| Aqua; Ethylhexyl Stearate; Sodium Hyaluronate Crosspolymer; Polyglyceryl-4 Diisostearate/Polyhydroxystearate/Sebacate; Sodium Isostearate (HyaCare® Filler CL, Evonik Industries) | 3,0% | 3,0% | |
| Demineralized Water | ad 100% | ad 100 % | ad 100% |
| Butylene Glycol | 5,0% | 5,0% | 5,0% |
| Tetrapeptide-21; Glycerin; Butylene Glycol; Aqua (TEGO® PEP 4-17, Evonik Industries) | 2,0% | 2,0% | 2,0% |
| Hydrolyzed Hyaluronic Acid (HyaCare® 50, Evonik Industries) | 0,1% | 0,1% | 0,1% |
| Xanthan Gum (Keltrol CG-SFT, CP Kelco) | 0,5% | 0,5% | 0,5% |
| Sodium Hydroxide (10% aq.) | 0,2% | 0,2% | 0,2% |
| Benzyl Alcohol, Benzoic Acid, Sorbic Acid (Rokonsal BSB-N, Ashland Specialty Ingredients) | 0,8% | 0,8% | 0,8% |
| Polyglutamic acid; Sclerotium Glucan; Betaine; Urea; Potassium Lactate (TEGO® Smooth Complex, Evonik Industries) | 3,0% | 3,0% | 3,0% |

**Rezepturen 20d, 20e und 20f: O/W Seren 2**

| **Rezeptur** | **20d** | **20e** | **20f** |
|---|---|---|---|
| Zusammensetzung aus Beispiel 14 | 3,0% | 3,0% | 2,0% |
| Cetearyl Glucoside (TEGO® Care CG 90, Evonik Nutrition & Care GmbH) | 0,5% | | |
| Polyglyceryl-3 Dicitrate/Stearate (TEGO® Care PSC 3, Evonik Nutrition & Care GmbH) | | | 1,5% |
| Glyceryl Stearate | 0,75% | 0,5% | 0,5% |
| Stearyl Alcohol | 0,75% | 0,5% | 0,5% |
| Caprylic/Capric Triglyceride | 2,0% | 2,0% | 2,0% |
| Oleyl Erucate (TEGOSOFT® OER, Evonik Nutrition & Care GmbH) | 2,0% | 2,0% | 2,0% |
| Isoamyl Cocoate (TEGOSOFT® AC, Evonik Nutrition & Care GmbH) | 3,0% | | |
| Persea Gratissima (Avocado) Oil | 1,0% | 1,0% | 1,0% |
| Aqua; Ethylhexyl Stearate; Sodium Hyaluronate Crosspolymer; Polyglyceryl-4 Diisostearate/Polyhydroxystearate/Sebacate; Sodium Isostearate (HyaCare® Filler CL, Evonik Industries) | | 3,0% | 3,0% |
| Demineralized Water | ad 100% | ad 100 % | ad 100 % |
| Butylene Glycol | 5,0% | 5,0% | 5,0% |
| Tetrapeptide-21; Glycerin; Butylene Glycol; Aqua (TEGO® PEP 4-17, Evonik Industries) | 2,0% | 2,0% | 2,0% |
| Hydrolyzed Hyaluronic Acid (HyaCare® 50, Evonik Industries) | 0,1% | 0,1% | 0,1% |
| Xanthan Gum (Keltrol CG-SFT, CP Kelco) | 0,5% | 0,5% | 0,5% |
| Sodium Hydroxide (10% aq.) | 0,2% | 0,2% | 0,2% |
| Benzyl Alcohol, Benzoic Acid, Sorbic Acid (Rokonsal BSB-N, Ashland Specialty Ingredients) | 0,8% | 0,8% | 0,8% |
| Polyglutamic acid; Sclerotium Glucan; Betaine; Urea; Potassium Lactate (TEGO® Smooth Complex, Evonik Industries) | 3,0% | | |

**Rezepturen 21a und 21b: O/W Blemish Balm Lotion**

| **Rezeptur** | **21a** | **21b** |
|---|---|---|
| Zusammensetzung aus Beispiel 14 | 4,00% | 3,00% |
| Polyglyceryl-3 Methylglucose Distearate (TEGO® Care 450, Evonik Nutrition & Care GmbH) | | 1,00% |
| Glyceryl Stearate | 0,75% | 0,75% |
| Stearyl Alcohol | 0,75% | 0,75% |
| Diethylhexyl Carbonate (TEGOSOFT® DEC, Evonik Nutrition & Care GmbH) | 7,40% | 7,40% |
| Aqua; Ethylhexyl Stearate; Sodium Hyaluronate Crosspolymer; Polyglyceryl-4 Diisostearate/Polyhydroxystearate/Sebacate; Sodium Isostearate (HyaCare® Filler CL, Evonik Industries) | 2,00% | 2,00% |
| Ethylhexyl Methoxycinnamate | 5,00% | 5,00% |
| Diethylamino Hydroxybenzoyl Hexyl Benzoate (Uvinul A Plus, BASF SE) | 3,00% | 3,00% |
| Phytosphingosine | 0,10% | 0,10% |
| Hydrolyzed Hyaluronic Acid (HyaCare® 50, Evonik Industries) | 0,10% | 0,10% |
| Tetrapeptide-21; Glycerin; Butylene Glycol; Aqua (TEGO® PEP 4-17, Evonik Industries) | 2,00% | 2,00% |
| Demineralized Water | ad 100% | ad 100% |
| Titanium Dioxide (Hombitan AC 360, Sachtleben) | 3,00% | 3,00% |
| Talc | 2,00% | 2,00% |
| Iron Oxide (Unipure Yellow LC 182, Sensient Technologies) | 0,36% | 0,36% |
| Iron Oxide (Unipure Red LC 381, Sensient Technologies) | 0,12% | 0,12% |
| Iron Oxide (Unipure Black LC 989, Sensient Technologies) | 0,08% | 0,08% |
| Isoamyl Cocoate (TEGOSOFT® AC, Evonik Nutrition & Care GmbH) | 4,44% | 4,44% |
| Phenoxyethyl Caprylate (TEGOSOFT® XC, Evonik Nutrition & Care GmbH) | 4,00% | 4,00% |
| Nylon-10/10 (TEGOLON® ECO 10-10, Evonik Nutrition & Care GmbH) | 3,00% | 3,00% |
| Glycerin | 3,00% | 3,00% |
| Xanthan Gum (Keltrol CG-SFT, CP Kelco) | 0,50% | 0,50% |
| Ethanol | 3,00% | 3,00% |
| Methylisothiazolinone, Methylparaben, Ethylparaben; Dipropylene Glycol (Microcare MEM, Thor) | 0,80% | 0,80% |

**Rezepturen 22a, 22b und 22c: Lotion für sensitive Haut**

| **Rezeptur** | **22a** | **22b** | **22c** |
|---|---|---|---|
| Zusammensetzung aus Beispiel 14 | 3,0% | 3,0% | 3,0% |
| Glyceryl Stearate | 0,5% | 1,0% | 0,5% |
| Cetearyl Alcohol | 1,0% | 1,0% | 1,0% |
| Butyrospermum Parkii (Shea) Butter | 3,0% | 3,0% | 3,0% |
| Caprylic/Capric Triglyceride | 5,0% | 5,0% | 5,0% |
| Isopropyl Palmitate (TEGOSOFT® P, Evonik Nutrition & Care GmbH) | 5,0% | 5,0% | 5,0% |
| Xanthan Gum (Keltrol CG-SFT, CP Kelco) | 0,2% | 0,2% | 0,2% |
| Glycerin | 5,0% | 5,0% | 5,0% |
| Urea | 10,0% | 15,0% | 20,0% |
| Demineralized Water | ad 100% | ad 100% | ad 100% |
| Phenoxyethanol, Ethylhexylglycerin (Euxyl PE 9010, Schülke & Mayr GmbH) | 0,7% | | |
| Sodium Hydroxide (10% aq.)(pH-Wert-Einstellung auf 5,0) | | q.s. | |
| Sodium Benzoate, Potassium Sorbate (Euxyl K 712, Schülke & Mayr GmbH) | | 1,2% | |
| Methylisothiazolinone, Methylparaben, Ethylparaben; Dipropylene Glycol (Microcare MEM, Thor) | | | 0,8% |

**Rezepturen 23a, 23b und 23c: Pflegende Lotion für trockene Haut 1**

| **Rezeptur** | **23a** | **23b** | **23c** |
|---|---|---|---|
| Zusammensetzung aus Beispiel 14 | 3,0% | 2,5% | 3,0% |
| Sodium Stearoyl Glutamate (Eumulgin SG, BASF SE) | | 1,0% | |
| Glyceryl Stearate | 1,0% | 1,0% | 1,5% |
| Cetearyl Alcohol | 1,0% | 1,0% | 1,5% |
| Cetyl Ricinoleate | 2,0% | 2,0% | 2,0% |
| Oleyl Erucate (TEGOSOFT® OER, Evonik Nutrition & Care GmbH) | 5,0% | 5,0% | 5,0% |
| Isoamyl Cocoate (TEGOSOFT® AC, Evonik Nutrition & Care GmbH) | 8,0% | 8,0% | 8,0% |
| Decyl Cocoate (TEGOSOFT® DC, Evonik Nutrition & Care GmbH) | 3,0% | 3,0% | 3,0% |
| Xanthan Gum (Keltrol CG-SFT, CP Kelco) | 0,2% | 0,2% | 0,2% |
| Glycerin | 5,0% | 5,0% | 5,0% |
| Urea | 10,0% | 10,0% | 15,0% |
| Demineralized Water | ad 100% | ad 100% | ad 100% |
| Methylisothiazolinone, Methylparaben, Ethylparaben; Dipropylene Glycol (Microcare MEM, Thor) | 0,8% | 0,8% | 0,8% |

**Rezepturen 23d, 23e und 23f: Pflegende Lotion für trockene Haut 2**

| **Rezeptur** | **23d** | **23e** | **23f** |
|---|---|---|---|
| Zusammensetzung aus Beispiel 14 | 2,5% | 3,0% | 3,0% |
| Polyglyceryl-6 Distearate | 0,5% | | |
| Stearic Acid | | | 0,5% |
| Glyceryl Stearate | 1,5% | 1,5% | 1,5% |
| Cetearyl Alcohol | 1,5% | 1,5% | 1,5% |
| Cetyl Ricinoleate | 2,0% | 2,0% | 2,0% |
| Oleyl Erucate (TEGOSOFT® OER, Evonik Nutrition & Care GmbH) | 5,0% | 5,0% | 5,0% |
| Isoamyl Cocoate (TEGOSOFT® AC, Evonik Nutrition & Care GmbH) | 8,0% | 8,0% | 8,0% |
| Decyl Cocoate (TEGOSOFT® DC, Evonik Nutrition & Care GmbH) | 3,0% | 3,0% | 3,0% |
| Xanthan Gum (Keltrol CG-SFT, CP Kelco) | 0,2% | 0,2% | 0,2% |
| Glycerin | 5,0% | 5,0% | 5,0% |
| Urea | 15,0% | 20,0% | 20,0% |
| Demineralized Water | ad 100% | ad 100% | ad 100% |
| Methylisothiazolinone, Methylparaben, Ethylparaben; Dipropylene Glycol (Microcare MEM, Thor) | 0,8% | 0,8% | 0,8% |

**Rezepturen 24a, 24b und 24c: Konservierungsmittelfreie Lotionen 1**

| **Rezeptur** | **24a** | **24b** | **24c** |
|---|---|---|---|
| Zusammensetzung aus Beispiel 14 | 3,0% | 2,0% | 3,0% |
| (AXOL® C 62, Evonik Nutrition & Care GmbH) | | 1,0% | |
| Glyceryl Stearate | 0,2% | 0,2% | 0,5% |
| Stearyl Alcohol | 0,2% | 0,2% | 0,5% |
| Prunus Amygdalus Dulcis (Sweet Almond) Oil | 10,0% | 10,0% | 10,0% |
| Isoamyl Cocoate (TEGOSOFT® AC, Evonik Nutrition & Care GmbH) | 6,6% | 6,6% | 6,0% |
| Glycerin | 4,0% | 4,0% | 4,0% |
| Demineralized Water | ad 100,0% | ad 100,0% | ad 100,0% |
| Caprylyl Glycol, Glycerin, Glyceryl Caprylate, Phenylpropanol (Dermosoft® LP, Evonik Dr. Straetmans GmbH) | 1,0% | 1,0% | |
| Methylpropanediol, Caprylyl Glycol, Phenylpropanol (Dermosoft® OMP, Evonik Dr. Straetmans GmbH) | | | 4,0% |
| Xanthan Gum (Keltrol CG-SFT, CP Kelco) | 0,5% | 0,5% | 0,5% |

| | | | |
|---|---|---|---|
| ⁴⁵⁾ Dermosoft® Octiol (Evonik Dr. Straetmans GmbHEvonik Dr. Straetmans GmbH) ⁴⁶⁾ 3,0g Natrium Hydroxid in 56,5g demineralisiertem Wasser lösen, 30,0g Glycerin und 10,0g Dermosoft® Octiol (Evonik Dr. Straetmans GmbH) zugeben, rühren bis Lösung klar ist. | | | |

**Rezepturen 24d, 24e und 24f: Konservierungsmittelfreie Lotionen 2**

| **Rezeptur** | **24d** | **24e** | **24f** |
|---|---|---|---|
| Zusammensetzung aus Beispiel 14 | 3,0% | 3,0% | 1,5% |
| Glyceryl Stearate SE | 0,5% | | |
| Polyglyceryl-3 Dicitrate/Stearate (TEGO® Care PSC 3, Evonik Nutrition & Care GmbH) | | | 1,5% |
| Glyceryl Stearate | 0,5% | 0,5% | 0,5% |
| Stearyl Alcohol | 0,5% | 0,5% | 0,5% |
| Prunus Amygdalus Dulcis (Sweet Almond) Oil | 10,0% | 10,0% | 10,0% |
| Isoamyl Cocoate (TEGOSOFT® AC, Evonik Nutrition & Care GmbH) | 6,0% | 6,0% | 6,0% |
| Glycerin | 4,0% | 4,0% | 4,0% |
| Demineralized Water | ad 100,0% | ad 100,0% | ad 100,0% |
| Methylpropanediol, Caprylyl Glycol, Phenylpropanol (Dermosoft® OMP, Evonik Dr. Straetmans GmbH) | 4,0% | | |
| Caprylyl Glycol (Dermosoft® Octiol, Evonik Dr. Straetmans GmbH) | | 0,4% | 0,4% |
| p-Anisic Acid-Lösung 10%ig (3,0g Natrium Hydroxid in 56,5g demineralisiertem Wasser lösen, 30,0g Glycerin und 10,0g Dermosoft® Octiol [Evonik Dr. Straetmans GmbH] zugeben und rühren, bis die Lösung klar ist) | | 2,0% | 2,0% |
| Citric Acid (10% aq.) (pH-Wert-Einstellung auf 6,0) | | q.s. | q.s. |
| Xanthan Gum (Keltrol CG-SFT, CP Kelco) | 0,5% | 0,5% | 0,5% |

**Rezeptur 25: W/O Lotion**

| **Rezeptur** | **25** |
|---|---|
| Zusammensetzung aus Beispiel 9 oder 10 | 2,0% |
| Bienenwachs | 0,5% |
| Castorwachs | 0,5% |
| Paraffinum Perliquidum | 10,5% |
| Decyl Cocoate (TEGOSOFT® DC, Evonik Nutrition & Care GmbH) | 8,0% |
| Tocopherylacetat | 0,5% |
| Cyclopentasiloxan | 6,0% |
| Natriumchlorid | 0,5% |
| Wasser | Ad 100% |
| Glycerin | 3,0% |
| Phenoxyethanol; Ethylhexylglycerin (Euxyl PE 9010, Schülke & Mayr GmbH) | 0,7% |
| Ethanol | 5,0% |

**Rezeptur 26: W/O Creme**

| **Rezeptur** | **26** |
|---|---|
| Zusammensetzung aus Beispiel 9 oder 10 | 2,0% |
| Mineralöl | 17,0% |
| Castorwachs | 0,4% |
| Mikrokristallines Wachs | 0,6% |
| Wasser | Ad 100% |
| Natriumchlorid | 0,5% |
| Harnstoff | 10,0% |
| Phenoxyethanol; Ethylhexylglycerin (Euxyl PE 9010, Schülke & Mayr GmbH) | 0,7% |

**Rezeptur 27: Quick-breaking Creme**

| **Rezeptur** | **27** |
|---|---|
| Zusammensetzung aus Beispiel 9 oder 10 | 0,8% |
| Cetyl Dimethicone (ABIL® Wax 9801, Evonik Nutrition & Care GmbH) | 1,6% |
| Diethylhexyl Carbonate (TEGOSOFT® DEC, Evonik Nutrition & Care GmbH) | 4,0% |
| Dimethicone (ABIL® 350, Evonik Nutrition & Care GmbH) | 1,0% |
| Cyclopentasiloxan | 4,0% |
| Magnesiumstearat | 0,3% |
| Wasser | Ad 100% |
| Propylenglykol | 5,0% |
| Natriumchlorid | 1,0% |
| Methylisothiazolinone, Methylparaben, Ethylparaben; Dipropylene Glycol (Microcare MEM, Thor) | 0,8% |

**Rezepturen 28a und 28b: Cooling Body Lotion**

| **Rezeptur** | **28a** | **28b** |
|---|---|---|
| Zusammensetzung aus Beispiel 9 oder 10 | 2,0% | 1,5% |
| Polyglyceryl-4 Diisostearate/ Polyhydroxystearate/Sebacate (ISOLAN® GPS, Evonik Nutrition & Care GmbH) | - | 0,5% |
| Castorwachs | 0,5% | 0,5% |
| Bienenwachs | 0,5% | 0,5% |
| Ethylhexyl Stearate (TEGOSOFT® OS, Evonik Nutrition & Care GmbH) | 10,0% | 10,0% |
| Diethylhexyl Carbonate (TEGOSOFT® DEC, Evonik Nutrition & Care GmbH) | 8,5% | 8,5% |
| Dimethicone (Belsil DM 5, Wacker Chemical Corp.) | 6,0% | 6,0% |
| Tocopherylacetate | 0,5% | 0,5% |
| Glycerin | 3,0% | 3,0% |
| Wasser | Ad 100% | Ad 100% |
| Natriumchlorid | 1,0% | 1,0% |
| Ethanol | 20,0% | 20,0% |

**Rezepturen 29a und 29b: W/O Creme basierend auf natürlichen Inhaltstoffen**

| **Rezeptur** | **29a** | **29b** |
|---|---|---|
| Zusammensetzung aus Beispiel 9 oder 10 | 3,0% | 2,5% |
| Diisostearyl Polyglyceryl-3 Dimer Dilinoleate (ISOLAN® PDI, Evonik Nutrition & Care GmbH) | - | 0,5% |
| Diethylhexyl Carbonate (TEGOSOFT® DEC, Evonik Nutrition & Care GmbH) | 7,0% | 7,0% |
| Oleyl Erucate (TEGOSOFT® OER, Evonik Nutrition & Care GmbH) | 3,0% | 3,0% |
| Mandelöl | 7,0% | 7,0% |
| Sheabutter | 2,0% | 2,0% |
| Cetyl Ricinoleate (TEGOSOFT® CR, Evonik Nutrition & Care GmbH) | 1,0% | 1,0% |
| Bienenwachs | 0,6% | 0,6% |
| Castorwax | 0,4% | 0,4% |
| Glycerin | 5,0% | 5,0% |
| Wasser | Ad 100% | Ad 100% |
| Magnesiumsulfat Heptahydrat | 1,5% | 1,5% |
| Sodium Benzoate, Potassium Sorbate (Euxyl K 712, Schülke & Mayr GmbH) | 0,5% | 0,5% |

**Rezepturen 30a und 30b: Kalt herstellbare Lotion**

| **Rezeptur** | **30a** | **30b** |
|---|---|---|
| Zusammensetzung aus Beispiel 9 oder 10 | 3.0% | 2.5% |
| Polyglyceryl-3 Oleate (ISOLAN® GO 33, Evonik Nutrition & Care GmbH) | - | 0.5% |
| Isoamyl Cocoate (TEGOSOFT® AC, Evonik Nutrition & Care GmbH) | 5.0% | 5.0% |
| Diethylhexyl Carbonate (TEGOSOFT® DEC, Evonik Nutrition & Care GmbH) | 12.0% | 12.0% |
| Phenoxyethyl Caprylate (TEGOSOFT® XC, Evonik Nutrition & Care GmbH) | 4.0% | 4.0% |
| Zinkstearate | 0.5% | 0.5% |
| Wasser | Ad 100% | Ad 100% |
| Glycerin | 3.0% | 3.0% |
| Natriumchlorid | 1.5% | 1.5% |
| Phenoxyethanol; Ethylhexylglycerin (Euxyl PE 9010, Schülke & Mayr GmbH) | 0.7% | 0.7% |

**Rezepturen 31a und 31b: Feuchtigkeitsspendende Lotion mit Harnstoff**

| **Rezeptur** | **31a** | **31b** |
|---|---|---|
| Zusammensetzung aus Beispiel 9 oder 10 | 2,0% | 1,5% |
| Cetyl PEG/PPG-10/1 Dimethicone (ABIL® EM 180, Evonik Nutrition & Care GmbH) | - | 0,5% |
| Mikrokristallines Wachs | 0,5% | 0,5% |
| Castorwachs | 0,5% | 0,5% |
| C12-15 Alkyl Benzoate | 7,5% | 7,5% |
| Oleyl Erucate (TEGOSOFT® OER, Evonik Nutrition & Care GmbH) | 5,0% | 5,0% |
| Ethylhexyl Palmitate (TEGOSOFT® OP, Evonik Nutrition & Care GmbH) | 5,0% | 5,0% |
| Caprylic/Capric Triglyceride | 5,0% | 5,0% |
| Glycerin | 3,0% | 3,0% |
| Harnstoff | 20,0% | 20,0% |
| Magnesiumsulfat Heptahydrat | 1,0% | 1,0% |
| Wasser | Ad 100% | Ad 100% |
| Phenoxyethanol; Ethylhexylglycerin (Euxyl PE 9010, Schülke & Mayr GmbH) | 0,70% | 0,70% |
| Parfum | 0,10% | 0,10% |

**Rezepturen 32a, 32b und 32c: W/O Lotion mit seidig-leichtem Hautgefühl**

| **Rezeptur** | **32a** | **32b** | **32c** |
|---|---|---|---|
| Zusammensetzung aus Beispiel 9 oder 10 | 2,5% | 2,0% | 2,0% |
| Cetyl PEG/PPG-10/1 Dimethicone (ABIL® EM 90, Evonik Nutrition & Care GmbH) | - | 0,5% | - |
| Bis-PEG/PPG-14/14 Dimethicone; Dimethicone (ABIL®, EM 97 S, Evonik Nutrition & Care GmbH) | - | - | 1,0% |
| Mikrokristallines Wachs | 0,1% | 0,1% | 0,1% |
| Castorwachs | 0,1% | 0,1% | 0,1% |
| Diethylhexyl Carbonate (TEGOSOFT® DEC, Evonik Nutrition & Care GmbH) | 11,8% | 11,8% | 11,8% |
| Myristyl Myristate (TEGOSOFT® MM, Evonik Nutrition & Care GmbH) | 1,0% | 1,0v | 1,0% |
| Dimethicone (Belsil DM 5, Wacker Chemical Corp.) | 8,0% | 8,0% | 8,0% |
| Dimethicone (ABIL® 350, Evonik Nutrition & Care GmbH) | 0,5% | 0,5% | 0,5% |
| Glycerin | 3,0% | 3,0% | 3,0% |
| Magnesiumsulfat Heptahydrat | 1,5% | 1,5% | 1,5% |
| Wasser | Ad 100% | Ad 100% | Ad 100% |
| Benzyl Alcohol; Ethylhexylglycerin; Tocopherol (Euxyl K 900, Schülke & Mayr GmbH) | 0,7% | 0,7% | 0,7% |

**Rezepturen 33a und 33b: Babypflege**

| **Rezeptur** | **33a** | **33b** |
|---|---|---|
| Zusammensetzung aus Beispiel 9 oder 10 | 3,0% | 2,0% |
| Paraffinum Liquidum; Petrolatum; Ozokerite; Glyceryl Oleate; Lanolin Alcohol (PROTEGIN ® XN, Evonik Nutrition & Care GmbH) | - | 1,0% |
| Castorwachs | 0,1% | 0,1% |
| Mikrokristallines Wachs | 0,1% | 0,1% |
| Oleyl Erucate (TEGOSOFT® OER, Evonik Nutrition & Care GmbH) | 1,0% | 1,0% |
| Isoamyl Cocoate (TEGOSOFT® AC, Evonik Nutrition & Care GmbH) | 3,8% | 3,8% |
| Ethylhexyl Palmitate (TEGOSOFT® OP, Evonik Nutrition & Care GmbH) | 1,0% | 1,0% |
| Mandelöl | 1,0% | 1,0% |
| Zinkoxid | 20,0% | 20,0% |
| Glycerin | 3,0% | 3,0% |
| Magnesiumsulfat Heptahydrat | 1,0% | 1,0% |
| Sodium Lactate; Sodium PCA; Glycine; Fructose; Urea; Niacinamide; Inositol; Sodium Benzoate; Lactic Acid (LACTIL, Evonik Nutrition & Care GmbH) | 5,0% | 5,0% |
| Betaine (TEGO® Natural Betaine, Evonik Nutrition & Care GmbH) | 3,0% | 3,0% |
| Wasser | Ad 100% | Ad 100% |
| Benzyl Alcohol; Ethylhexylglycerin; Tocopherol (Euxyl K 900, Schülke & Mayr GmbH) | 0,7% | 0,7% |

**Rezepturen 34a und 34b: Fußpflege**

| **Rezeptur** | **34a** | **34b** |
|---|---|---|
| Zusammensetzung aus Beispiel 9 oder 10 | 3,0% | 2,5% |
| Petrolatum; Ozokerite; Hydrogenated Castor Oil; Glyceryl Isostearate; Polyglyceryl-3 Oleate (PROTEGIN ® W, Evonik Nutrition & Care GmbH) | - | 0,5% |
| Castorwachs | 0,1% | 0,1% |
| Mikrokristallines Wachs | 0,1% | 0,1% |
| Diethylhexyl Carbonate (TEGOSOFT® DEC, Evonik Nutrition & Care GmbH) | 9,0% | 9,0% |
| Ethylhexyl Palmitate (TEGOSOFT® OP, Evonik Nutrition & Care GmbH) | 9,0% | 9,0% |
| Stearyl Heptanoate (TEGOSOFT® SH, Evonik Nutrition & Care GmbH) | 8,8% | 8,8% |
| Glycerin | 3,0% | 3,0% |
| Magnesiumsulfat Heptahydrat | 1,0% | 1,0% |
| Ceramide NP; Ceramide AP; Ceramide EOP; Phytosphingosine; Cholesterol; Sodium Lauroyl Lactylate; Carbomer; Xanthan Gum (SK-INFLUX V, Evonik Nutrition & Care GmbH) | 5,0% | 5,0% |
| Betaine (TEGO® Natural Betaine, Evonik Nutrition & Care GmbH) | 3,0% | 3,0% |
| Wasser | Ad 100% | Ad 100% |
| Benzyl Alcohol; Ethylhexylglycerin; Tocopherol (Euxyl K 900, Schülke & Mayr GmbH) | 0,7% | 0,7% |

**Rezepturen 35a und 35b: Sonnenschutzlotion SPF 30 UVA mit Insektenschutz**

| **Rezeptur** | **35a** | **35b** |
|---|---|---|
| Zusammensetzung aus Beispiel 9 oder 10 | 3,0% | 2,0% |
| Polyglyceryl-2 Dipolyhydroxystearate (Dehymuls PGPH, BASF SE) | - | 1,0% |
| Oleyl Erucate (TEGOSOFT® OER, Evonik Nutrition & Care GmbH) | 1,5% | 1,5% |
| Diethylhexyl Carbonate (TEGOSOFT® DEC, Evonik Nutrition & Care GmbH) | 1,5% | 1,5% |
| Diethylamino Hydroxybenzoyl Hexyl Benzoate (Uvinul A Plus, BASF SE) | 5,4% | 5,4% |
| Ethylhexyl Methoxycinnamate | 10,0% | 10,0% |
| Octocrylene | 2,0% | 2,0% |
| Polyacrylamide; C13-14 Isoparaffin; Laureth-7 (Sepigel 305, Seppic) | 2,1% | 2,1% |
| Ethyl Butylacetylamin opropionate (R3535, Merck KGaA) | 4,0% | 4,0% |
| Tocopheryl Acetat | 0,5% | 0,5% |
| Glycerin | 3,0% | 3,0% |
| Ethanol | 0,5% | 0,5% |
| Magnesiumsulfat Heptahydrat | 1,0% | 1,0% |
| Wasser | Ad 100% | Ad 100% |
| Benzyl Alcohol; Ethylhexylg lycerin; Tocopherol (Euxyl K 900, Schülke & Mayr GmbH) | 0,7% | 0,7% |
| Parfum | 0,1% | 0,1% |

**Rezepturen 36a und 36b: Sonnenschutzlotion SPF 30 UVA nach Ecocertkriterien**

| **Rezeptur** | **36a** | **36b** |
|---|---|---|
| Zusammensetzung aus Beispiel 9 oder 10 | 3,0% | 2,0% |
| Polyglyceryl-3 Polyricinoleate (Cithrol PG3PR, (Croda Int. Plc) | - | 1,0% |
| Isoamyl Cocoate (TEGOSOFT® AC, Evonik Nutrition & Care GmbH) | 2,0% | 2,0% |
| Decyl Cocoate (TEGOSOFT® DC, Evonik Nutrition & Care GmbH) | 10,0% | 10,0% |
| Isopropyl Palmitate (TEGOSOFT® P, Evonik Nutrition & Care GmbH) | 10,0% | 10,0% |
| Zinc Oxide (Zinc Oxide Pl, Symrise) | 16,0% | 16,0% |
| Titanium Dioxide [nano]; Alumina; Stearic Acid (Eusolex T-S, Merck KGaA) | 9,0% | 9,0% |
| Wasser | Ad 100% | Ad 100% |
| Glycerin | 3,0% | 3,0% |
| Magnesiumsulfat Heptahydrat | 1,0% | 1,0% |
| Sodium Benzoate, Potassium Sorbate (Euxyl K 712, Schülke & Mayr GmbH) | 0,5% | 0,5% |

**Rezepturen 37a, 37b, 37c und 37d: Sonnenschutzspray SPF 30 UVA**

| **Rezeptur** | **37a** | **37b** | **37c** | **37d** |
|---|---|---|---|---|
| Zusammensetzung aus Beispiel 9 oder 10 | 3,0% | 2,0% | 3,0% | 2,0% |
| Cetyl PEG/PPG-10/1 Dimethicone (ABIL® EM 90, Evonik Nutrition & Care GmbH) | - | 1,0% | - | - |
| Polyglyceryl-4 Diisostearate/Polyhydroxystearate/Sebacate (ISOLAN® GPS, Evonik Nutrition & Care GmbH) | - | - | - | 1,0% |
| Diethylhexyl Carbonate (TEGOSOFT® DEC, Evonik Nutrition & Care GmbH) | 13,0% | 13,0% | 11,3% | 11,3% |
| C12-15 Alkyl Benzoate | 13,0% | 13,0% | 11,3% | 11,3% |
| Bis-Ethylhexyloxyphenol Methoxyphenyl Triazine (Tinosorb S, BASF SE) | 1,0% | 1,0% | 1,5% | 1,5% |
| Butyl Methoxydibenzoylmethane | - | - | 3,0% | 3,0% |
| Ethylhexyl Methoxycinnamate | 5,0% | 5,0% | - | - |
| Octocrylene | - | - | 6,0% | 6,0% |
| Homosalate | - | - | 4,0% | 4,0% |
| Diethylamino Hydroxybenzoyl Hexyl Benzoate (Uvinul A Plus, BASF SE) | 5,0% | 5,0% | - | - |
| Wasser | Ad 100% | Ad 100% | Ad 100% | Ad 100% |
| Glycerin | 3,0% | 3,0% | 3,0% | 3,0% |
| Magnesiumsulfat Heptahydrat | 1,0% | 1,0% | 1,0% | 1,0% |
| UV-Filterlösung (20 % Phenylbenzimidazole Sulfonic Acid (Eusolex 232, Merck KGaA, 8,8% Tris (hydroxymethyl)-aminomethan, Wasser ad 100%) | 15,0% | 15,0% | 15,0% | 15,0% |
| Benzyl Alcohol; Ethylhexylglycerin; Tocopherol (Euxyl K 900, Schülke & Mayr GmbH) | 0,7% | 0,7% | 0,7% | 0,7% |

**Rezepturen 38a, 38b, 38c und 38d: Sonnenschutzlotion SPF 50 UVA**

| **Rezeptur** | **38a** | **38b** | **38c** | **38d** |
|---|---|---|---|---|
| Zusammensetzung aus Beispiel 9 oder 10 | 3,0% | 2,5% | 3,0% | 2,5% |
| Cetyl PEG/PPG-10/1 Dimethicone (ABIL® EM 90, Evonik Nutrition & Care GmbH) | | 1,0% | | 1,0% |
| Mikrokristallines Wachs | 0,3% | 0,3% | 0,3% | 0,3% |
| Castorwachs | 0,3% | 0,3% | 0,3% | 0,3% |
| Diethylhexyl Carbonate (TEGOSOFT® DEC, Evonik Nutrition & Care GmbH) | 2,4% | 2,4% | - | - |
| Phenoxyethyl Caprylate (TEGOSOFT® XC, Evonik Nutrition & Care GmbH) | - | - | 3,9% | 3,9% |
| Bis-Ethylhexyloxyphenol Methoxyphenyl Triazine (Tinosorb S, BASF SE) | 6,0% | 6,0% | - | - |
| Diethylamino Hydroxybenzoyl Hexyl Benzoate (Uvinul A Plus, BASF SE) | 7,0% | 7,0% | 5,0% | 5,0% |
| Butyl Methoxydibenzoylmethane | - | - | 4,5% | 4,5% |
| Ethylhexyl Salicylate | 3,0% | 3,0% | 5,0% | 5,0% |
| Ethylhexyl Methoxycinnamate | 7,0% | 7,0% | - | - |
| Octocrylene | - | - | 9,0% | 9,0% |
| Homosalate | 3,0% | 3,0% | 5,0% | 5,0% |
| Ethylhexyl Triazone (Uvinul T 150, BASF SE) | 1,0% | 1,0% | 2,0% | 2,0% |
| Titanium Dioxide; Silica; Dimethicone (Parsol TX (DSM Nutritional Products Llc.) | 2,0% | 2,0% | 2,0% | 2,0% |
| Wasser | Ad 100% | Ad 100% | Ad 100% | Ad 100% |
| Glycerin | 3,0% | 3,0% | 3,0% | 3,0% |
| Magnesiumsulfat Heptahydrat | 1,5% | 1,5% | 1,5% | 1,5% |
| Benzyl Alcohol; Ethylhexylglycerin; Tocopherol (Euxyl K 900, Schülke & Mayr GmbH) | 0,7% | 0,7% | 0,7% | 0,7% |

**Rezepturen 39a und 39b: Sonnenschutzlotion SPF 50 nach FDA-Kriterien**

| **Rezeptur** | **39a** | **39b** |
|---|---|---|
| Zusammensetzung aus Beispiel 9 oder 10 | 2,0% | 1,5% |
| Lauryl PEG-10 Tris(Trimethylsiloxy)silylethyl Dimethicone (ES-5300 Formulation Aid, Dow Corning Corp.) | - | 0,5% |
| Ethylhexyl Methoxycinnamate; Diethylamino Hydroxybenzoyl Hexyl Benzoate (Uvinul A+B, BASF SE) | 7,5% | 7,5% |
| Ethylhexyl Salicylate | 5,0% | 5,0% |
| Homosalate | 15,0% | 15,0% |
| Butyl Methoxydibenzoylmethane | 3,0% | 3,0% |
| Benzophenone-3 | 6,0% | 6,0% |
| Octocrylene | 10,0% | 10,0% |
| Triisostearin | 2,0% | 2,0% |
| Mikrokristallines Wachs | 1,2% | 1,2% |
| Castorwachs | 0,8% | 0,8% |
| Cetyl Dimethicone (ABIL® Wax 9801, Evonik Nutrition & Care GmbH) | 2,0% | 2,0% |
| Diethylhexyl Carbonate (TEGOSOFT® DEC, Evonik Nutrition & Care GmbH) | 2,0% | 2,0% |
| Wasser | Ad 100% | Ad 100% |
| Natriumchlorid | 1,0% | 1,0% |
| Ethylendiamintetraessigsäure | 0,1% | 0,1% |
| Propylenglycol | 3,0% | 3,0% |
| Phenoxyethanol; Ethylhexylglycerin (Euxyl PE 9010, Schülke & Mayr GmbH) | 0,7% | 0,7% |

**Rezepturen 40a, 40b, 40c, 40d, 40e und 40f: Foundation**

| **Rezeptur** | **40a** | **40b** | **40c** | **40d** | **40e** | **40f** |
|---|---|---|---|---|---|---|
| Zusammensetzung aus Beispiel 9 oder 10 | 4,5% | 2,5% | 3,0% | 2,5% | 2,0% | 2.0% |
| Bis-(Glyceryl/Lauryl) Glyceryl Lauryl Dimethicone; Caprylic/Capric Triglyceride (ABIL® EM 120, Evonik Nutrition & Care GmbH) | - | 2,0% | - | - | - | - |
| Polyglyceryl-4 Isostearate (ISOLAN® GI 34, Evonik Nutrition & Care GmbH) | - | - | 1,0% | - | - | - |
| Cetyl Diglyceryl Tris(Trimethylsiloxy)silylethyl Dimethicone (DC-5600, Dow Corning Corp.) | - | - | - | 1,0% | - | - |
| Lauryl Polyglyceryl-3 Polydimethylsiloxyethyl Dimethicone (KF-6105, Shin-Etsu Chemical Co.) | - | - | - | - | 1,0% | - |
| Polyglyceryl-4 Isostearate; Cetyl PEG/PPG-10/1 Dimethicone; Hexyl Laurate (ABIL®® WE 09, Evonik Nutrition & Care GmbH) | - | - | - | - | - | 2,0% |
| Isoamyl Cocoate (TEGOSOFT® AC, Evonik Nutrition & Care GmbH) | 10,8% | 10,8% | 10,8% | 10,8% | 10,8% | 10,8% |
| Oleyl Erucate (TEGOSOFT® OER, Evonik Nutrition & Care GmbH) | 8,0% | 8,0% | 8,0% | 8,0% | 8,0% | 8,0% |
| Titanium Dioxide, Alumina, Triethoxycaprylylsilane (Hombitan AC360, Sachtleben) | 4,0% | 4,0% | 4,0% | 4,0% | 4,0% | 4,0% |
| Iron Oxides (Sicovit Braun 70 E 172, Rockwood) | 2,1% | 2,1% | 2,1% | 2,1% | 2,1% | 2,1% |
| Nylon-12 (TEGOLON® 12-20, Evonik Nutrition & Care GmbH) | 2,0% | 2,0% | 2,0% | 2,0% | 2,0% | 2,0% |
| Cylcopentasiloxane | 3,5% | 3,5% | 3,5% | 3,5% | 3,5% | 3,5% |
| Disteardimonium Hectorite (Bentone 38 V CG, Elementis) | 1,0% | 1,0% | 1,0% | 1,0% | 1,0% | 1,0% |
| Propylencarbonat | 0,5% | 0,5% | 0,5% | 0,5% | 0,5% | 0,5% |
| Wasser | Ad 100% | Ad 100% | Ad 100% | Ad 100% | Ad 100% | Ad 100% |
| Magnesiumsulfat Hepathydrat | 1,5% | 1,5% | 1,5% | 1,5% | 1,5% | 1,5% |
| Glycerin | 5,0% | 5,0% | 5,0% | 5,0% | 5,0% | 5,0% |
| Creatine (TEGO® Cosmo C 100, Evonik Nutrition & Care GmbH) | 0,5% | 0,5% | 0,5% | 0,5% | 0,5% | 0,5% |
| Ceteareth-25; Glycerin; Cetyl Alcohol; Behenic Acid; Cholesterol; Ceramide EOP; Ceramide EOS; Ceramide NP; Ceramide NS; Ceramide AP; Caprooyl Phytospingosine; Caprooyl Sphingosine (SKINMIMICS, Evonik Nutrition & Care GmbH) | 1,5% | 1,5% | 1,5% | 1,5% | 1,5% | 1,5% |
| Benzyl Alcohol; Ethylhexylglycerin; Tocopherol (Euxyl K 900, Schülke & Mayr GmbH) | 0,7% | 0,7% | 0,7% | 0,7% | 0,7% | 0,7% |

**Rezepturen 41a, 41b, 41c, 41d, 41e, 41f und 41g: CC (Color Control) Fluid**

| **Rezeptur** | **41a** | **41b** | **41c** | **41d** | **41e** | **41f** | **41g** |
|---|---|---|---|---|---|---|---|
| Zusammensetzung aus Beispiel 9 oder 10 | 3,0% | 2,0% | 2,5% | 3,0% | 2,5% | 2,0% | 1,0% |
| Polyglyceryl-4 Diisostearate/ Polyhydroxystearate/Sebacat e (ISOLAN® GPS, Evonik Nutrition & Care GmbH) | - | 1,0% | - | - | - | - | - |
| Sorbitan Oleate (TEGO® SMO V, Evonik Nutrition & Care GmbH) | - | - | 0,5% | - | - | - | - |
| PEG-30 Dipolyhydroxystearate (Arlacel P135, Croda) | - | - | - | 1,0% | - | - | - |
| Polyglyceryl-3 Diisostearate (Lameform TGI, BASF SE) | - | - | - | - | 1,5% | - | - |
| Glyceryl Oleate, Polyglyceryl-3 Polyricinoleate, Olea Europaea (Olive) Oil Unsaponifiables (Plantasens Natural Emulsifier CP5, Clariant) | - | - | - | - | - | 1,0% | - |
| Lauryl PEG-9 Polydimethylsiloxyethyl Dimethicone (KF-6038, Shin-Etsu Chemical Co.) | - | - | - | - | - | - | 1,0% |
| Ethylhexyl Methoxycinnamate; Diethylamino Hydroxybenzoyl Hexyl Benzoate (Uvinul A+B, BASF SE) | 10,0% | 10,0% | 10,0% | 10,0% | 10,0% | 10,0% | 10,0% |
| Cylcopentasiloxane | 3,5% | 3,5% | 3,5% | 3,5% | 3,5% | 3,5% | 3,5% |
| Disteardimonium Hectorite (Bentone 38 V CG, Elementis) | 1,0% | 1,0% | 1,0% | 1,0% | 1,0% | 1,0% | 1,0% |
| Propylencarbonat | 0,5% | 0,5% | 0,5% | 0,5% | 0,5% | 0,5% | 0,5% |
| Titanium Dioxide, Alumina, Trieth oxycaprylylsila ne (Hombitan AC360, Sachtleben) | 4,0% | 4,0% | 4,0% | 4,0% | 4,0% | 4,0% | 4,0% |
| Talc | 2,0% | 2,0% | 2,0% | 2,0% | 2,0% | 2,0% | 2,0% |
| Iron Oxides; Trieth oxycaprylylsila ne (Unipure Yellow LC 182 AS-EM, Sensient) | 0,4% | 0,4% | 0,4% | 0,4% | 0,4% | 0,4% | 0,4% |
| Iron Oxides; Trieth oxycaprylylsila ne (Unipure Red LC 381 AS-EM, Sensient) | 0,12% | 0,12% | 0,12% | 0,12% | 0,12% | 0,12% | 0,12% |
| Iron Oxides; Trieth oxycaprylylsila ne (Unipure Black LC 989 AS-EM, Sensient) | 0,08% | 0,08% | 0,08% | 0,08% | 0,08% | 0,08% | 0,08% |
| Diethylhexyl Carbonate (TEGOSOFT® DEC, Evonik Nutrition & Care GmbH) | 4,0% | 4,0% | 4,0% | 4,0% | 4,0% | 4,0% | 4,0% |
| C12-15 Alkyl Benzoate | 4,0% | 4,0% | 4,0% | 4,0% | 4,0% | 4,0% | 4,0% |
| Isopropyl Palmitate (TEGOSOFT® P, Evonik Nutrition & Care GmbH) | 4,0% | 4,0% | 4,0% | 4,0% | 4,0% | 4,0% | 4,0% |
| Nylon-12 (TEGOLON® 12-20, Evonik Nutrition & Care GmbH) | 2,0% | 2,0% | 2,0% | 2,0% | 2,0% | 2,0% | 2,0% |
| Wasser | Ad 100% | Ad 100% | Ad 100% | Ad 100% | Ad 100% | Ad 100% | Ad 100% |
| Glycerin | 5,0% | 5,0% | 5,0% | 5,0% | 5,0% | 5,0% | 5,0% |
| Natriumchlorid | 1,5% | 1,5% | 1,5% | 1,5% | 1,5% | 1,5% | 1,5% |
| Tetrapeptide-30; Glycerin (TEGO® Pep 4-Even, Evonik Nutrition & Care GmbH) | 0,3% | 0,3% | 0,3% | 0,3% | 0,3% | 0,3% | 0,3% |
| Phenoxyethanol; Methylparaben; Ethylparaben; Propylparaben (Phenonip XB, Clariant) | 0,7% | 0,7% | 0,7% | 0,7% | 0,7% | 0,7% | 0,7% |

**Rezepturen 42a, 42b, 42c und 42d: APIDeo Spray bzw. Aerosolspray**

| **Rezeptur** | **42a** | **42b** | **42c** | **42d** |
|---|---|---|---|---|
| Zusammensetzung aus Beispiel 9 oder 10 | 3,0% | 2,0% | 3,0% | 2,0% |
| Polyglyceryl-4 Diisostearate/ Polyhydroxystearate/Sebac ate (ISOLAN® GPS, Evonik Nutrition & Care GmbH) | - | 1,0% | - | 1,0% |
| Isopropyl Palmitate (TEGOSOFT® P, Evonik Nutrition & Care GmbH) | 20,0% | 20,0% | 20,0% | 20,0% |
| Diethylhexyl Carbonate (TEGOSOFT® DEC, Evonik Nutrition & Care GmbH) | 7,0% | 7,0% | 7,0% | 7,0% |
| Wasser | Ad 100% | Ad 100% | Ad 100% | Ad 100% |
| Glycerin | 2,0% | 2,0% | 2,0% | 2,0% |
| Aluminum Chlorohydrate (50% aq.; Locron LIC, Clariant AG) | 30,0% | 30,0% | 30,0% | 30,0% |
| Parfum | 1,0% | 1,0% | 1,0% | 1,0% |
| Treibgas | - | - | Emulsionen 42c und 42d mit Treibgas im Massenverhältnis 5:2 mischen | |

**Rezepturen 43a, 43b, 43c und 43d: Sonnenschutzaerosol SPF 50 UVA**

| **Rezeptur** | **43a** | **43b** | **43c** | **43d** |
|---|---|---|---|---|
| Zusammensetzung aus Beispiel 9 oder 10 | 4,0% | 4,0% | 4,0% | 4,0% |
| Cetyl PEG/PPG-10/1 Dimethicone (ABIL® EM 90, Evonik Nutrition & Care GmbH) | - | - | 1,0% | 1,0% |
| C12-15 Alkyl Benzoate | 10,0% | 8,0% | 10,0% | 8,0% |
| Diethylhexyl Carbonate (TEGOSOFT® DEC, Evonik Nutrition & Care GmbH) | 13,0% | 10,0% | 13,0% | 10,0% |
| Bis-Ethylhexyloxyphenol Methoxyphenyl Triazine (Tinosorb S, BASF SE) | 4,0% | 4,0% | 4,0% | 4,0% |
| Diethylamino Hydroxybenzoyl Hexyl Benzoate (Uvinul A Plus, BASF SE) | 5,0% | 5,0% | 5,0% | 5,0% |
| Ethylhexyl Salicylate | 5,0% | 5,0% | 5,0% | 5,0% |
| Ethylhexyl Methoxycinnamate | 4,0% | 4,0% | 4,0% | 4,0% |
| Wasser | Ad 100% | Ad 100% | Ad 100% | Ad 100% |
| Glycerin | 3,0% | 3,0% | 3,0% | 3,0% |
| UV-Filterlösung (20 % Phenylbenzimidazole Sulfonic Acid (Eusolex 232, Merck KGaA, 8,8% Tris (hydroxymethyl)-aminomethan, Wasser ad 100%) | 20,0% | 20,0% | 20,0% | 20,0% |
| Magnesiumsulfat Heptahydrat | 1,0% | 1,0% | 1,0% | 1,0% |
| Benzyl Alcohol; Ethylhexylglycerin; Tocopherol (Euxyl K 900, Schülke & Mayr GmbH) | 0,7% | 0,7% | 0,7% | 0,7% |
| Emulsionen 43a, 43b, 43c und 43d mit Treibgas im Massenverhältnis 2:1 mischen | | | | |

**Rezeptur 44: Creme-Dusche**

| Water | ad 100,0% |
|---|---|
| Zusammensetzung aus Beispiel 4, 8, 15 oder 16 | 1,5% |
| Sodium Laureth Sulfate (Texapon NSO, BASF, 28%) | 25,0% |
| Coco-Glucoside (Plantacare 818 UP, BASF, 51%) | 8,0% |
| Cocamidopropyl Betaine (TEGO® Betain F 50, Evonik, 38%) | 8,0% |
| PEG-18 Glyceryl Oleate/Cocoate (ANTIL® 171, Evonik) | 1,5% |
| Sorbitan Sesquicaprylate (ANTIL® Soft SC, Evonik) | 0,8% |
| Glyceryl Oleate (TEGIN ® O V, Evonik) | 0,8% |
| Perfume Spicy Herbs (IFF) | 0,2% |
| Polyglyceryl-4 Caprate (TEGOSOFT® PC 41, Eovnik) | 0,6% |
| Helianthus Annuus Seed Oil (AEC Sunflower Oil, A & E Connock, Perfumery & Cosmetics Ltd.) | 0,2% |
| Linalool (Lipofresh, Lipo Chemicals, Inc.) | 0,1% |
| Coumarin (Rhodiascent extra pure, Solvay Rhodia) | 0,1% |
| Glycerin (Glycerol EP, vegetable, Spiga Nord) | 0,4% |
| Hydroxypropyl Methylcellulose (TEGOCEL ® HPM 50, Evonik) | 0,2% |
| Glycol Distearate (TEGIN ® G 1100 Pellets, Evonik) | 0,4% |
| Sodium Chloride | 0,5% |
| Hydroxypropyl Guar Hydroxypropyltrimonium Chloride (Jaguar C-162, Solvay Rhodia) | 0,2% |
| Tocopherol (Euxyl K 700, Schülke & Mayr GmbH) | 0,1% |
| Disodium EDTA (Dissolvine NA-2-P, AkzoNobel) | 0,1% |
| Preservative | q.s. |
| Citric Acid | ad pH 5,2 |

**Rezeptur 45: Körpershampoo**

| | | |
|---|---|---|
| Phase A | Zusammensetzung aus Beispiel 4, 8, 15 oder 16 | 5,0% |
| | Lavandula Angustifolia (Lavender) Oil (AEC Lavender Oil, A&E Connock Ltd.) | 0,2% |
| | Perfume | 0,1% |
| Phase B | Sodium Cocoamphoacetate (REWOTERIC® AM C, Evonik, 32%) | 10,0% |
| Phase C | Water | ad 100,0% |
| | Xanthan Gum (Keltrol CG-SFT, CP Kelco) | 1,2% |
| Phase D | Sodium Lauroyl Methyl Isethionate (Iselux, Innospec Active Chemicals) | 4,5% |
| | Capryl/Capramidopropyl Betaine (TEGO® Betaine 810, Evonik, 38%) | 4,5% |
| | Citric Acid | 1,2% |
| Phase E | Water | 10,0% |
| | Polyquaternium-7 (Merquat 550, Nalco) | 0,4% |
| | Preservative | q.s. |

**Rezeptur 46: Shampoo**

| | | |
|---|---|---|
| Phase A | Zusammensetzung aus Beispiel 4, 8, 15 oder 16 | 3,5% |
| | Isopropyl Myristate (TEGOSOFT® M, Evonik) | 0,2% |
| | Perfume | 0,1% |
| Phase B | Water | ad 100,0% |
| Phase C | Sodium Lauryl Sulfate (Texapon LS 35, BASF, 30%) | 28,0% |
| Phase D | Cocamidopropyl Betaine (TEGO® Betain F 50, Evonik, 38%) | 9,0% |
| Phase E | Cocamide MEA (REWOMID® C 212, Evonik) | 2,0% |
| | Xanthan Gum (Keltrol CG-SFT, CP Kelco) | 0,3% |
| | Water | 10,0% |
| Phase F | Water | 10,0% |
| | Polyquaternium-10 (Polymer JR 400, Amerchol) | 0,2% |
| Phase G | Citric Acid | ad pH 5,0 |
| Phase H | Preservative | q.s. |

**Rezeptur 47: Shampoo**

| Water | ad 100,0% |
|---|---|
| Zusammensetzung aus Beispiel 4, 8, 15 oder 16 | 2,5% |
| Zusammensetzung aus Beispiel 14 | 1,5% |
| Cocamidopropyl Betaine (TEGO® Betain F 50, Evonik, 38%) | 22,0% |
| Lauryl Glucoside (Plantacare 1200 UP, BASF, 50%) | 6,0% |
| Sodium Cocoyl Glutamate (Plantapon ACG HC, BASF) | 1,5% |
| Sodium Cocoyl Glycinate (Hostapon SG, Clariant) | 0,8% |
| Zinc Pyrithione (Microcare ZP, Thor) | 0,1% |
| PEG-120 Methyl Glucose Dioleate (ANTIL® 120 Plus, Evonik) | 0,4% |
| Sodium Chloride | 0,5% |
| Isostearamide MIPA; Glyceryl Laurate (ANTIL® SPA 80, Evonik) | 0,5% |
| Xanthan Gum (Keltrol CG-SFT, CP Kelco) | 0,3% |
| Hydroxypropyl Guar Hydroxypropyltrimonium Chloride (Jaguar C-162, Solvay Rhodia) | 0,3% |
| Quaternium-80 (ABIL® Quat 3272, Evonik) | 0,4% |
| Palmitamidopropyltrimonium Chloride (VARISOFT® PATC, Evonik) | 0,4% |
| Argania Spinosa Oil (Argan Oil, DSM Nutritional Products Ltd.) | 0,1% |
| Glycerin (Glycerol EP, vegetable, Spiga Nord) | 0,6% |
| Tetrasodium EDTA (Versene 100, The Dow Chemical Company) | 0,1% |
| Caffeine (Merck KGaA /EMD Chemicals, Inc.) | 0,1% |
| Hydrolyzed Wheat Protein (Gluadin WLM, BASF) | 0,1% |
| Limonene (Dipentene No. 122, Hercules Inc.) | 0,1% |
| Citric Acid | ad pH 5,5 |
| Perfume | 0,2% |
| Preservative | q.s. |

**Rezeptur 48: Liquid Soap**

| Water | ad 100% |
|---|---|
| Glycerin (Glycerol EP, vegetable, Spiga Nord) | 4,0% |
| Alcohol | 4,0% |
| Sodium Coco-Sulfate (Texapon HC G, BASF) | 3,0% |
| Lauryl Glucoside (Plantacare 1200 UP, BASF, 50%) | 6,0% |
| Zusammensetzung aus Beispiel 4, 8, 15 oder 16 | 2,0% |
| Zusammensetzung aus Beispiel 17 | 1,0% |
| Xanthan Gum (Keltrol CG-SFT, CP Kelco) | 1,5% |
| Mangifera Indica (Mango) Fruit Extract (Mango Extract, Draco Natural Products) | 0,5% |
| Limonene (Dipentene No. 122, Hercules Inc.) | 0,1% |
| Linalool (Lipofresh, Lipo Chemicals, Inc.) | 0,1% |
| Citric Acid | ad pH 4,9 |
| Preservative | q.s. |
| Dyes | q.s. |

**Rezeptur 49: Cream Soap**

| Water | ad 100% |
|---|---|
| Propylene Glycol (Euxyl K 320, Schülke & Mayr GmbH) | 2,0% |
| Coco-Glucoside (Plantacare 818 UP, BASF, 51%) | 10,0% |
| Glycerin (Glycerol EP, vegetable, Spiga Nord) | 5,0% |
| Zusammensetzung aus Beispiel 4, 8, 15 oder 16 | 2,5% |
| Disodium Cocoyl Glutamate (Planatpon ACG LC, BASF) | 2,5% |
| Xanthan Gum (Keltrol CG-SFT, CP Kelco) | 1,2% |
| Stearic Acid (Pristerene 4922, Croda Europe, Ltd.) | 1,2% |
| Citric Acid | ad pH 5,5 |
| Olea Europaea Fruit Oil (Cropure Olive, Croda Europe, Ltd.) | 0,2% |
| Glyceryl Oleate (TEGIN ® O V, Evonik) | 1,0% |
| Sodium Cocoyl Glutamate (Plantapon ACG HC, BASF) | 0,8% |
| Tetrasodium EDTA (Versene 100, The Dow Chemical Company) | 0,2% |
| Perfume | 0,1% |
| Preservative | q.s. |
| Dyes | q.s. |

**Rezeptur 50: Oil Bath**

| Water | ad 100,0% |
|---|---|
| Glycine Soja Oil (Cropure Soybean, Croda Europe, Ltd.) | 20,0% |
| Zusammensetzung aus Beispiel 4, 8, 15 oder 16 | 12,0% |
| Polyglyceryl-3 Palmitate (Dermofeel® PP, Evonik Dr. Straetmans) | 4,5% |
| Glyceryl Caprylate (Dermosoft® GMCY, Evonik Dr. Straetmans) | 3,0% |
| Simmondsia Chinensis Seed Oil (AEC Jojoba Oil Refined, A & E Connock, Perfumery & Cosmetics Ltd.) | 1,2% |
| Prunus Amygdalus Dulcis (Sweet Almond) Oil (Cropure Almond, Croda Europe, Ltd.) | 1,0% |
| Triticum Vulgare Germ Oil (Cropure Wheatgerm, Croda Europe, Ltd.) | 0,5% |
| Tocopherol (Euxyl K 700, Schülke & Mayr GmbH) | 0,2% |
| Limonene (Dipentene No. 122, Hercules Inc.) | 0,1% |
| Citral | 0,1% |
| Preservative | q.s. |
| Dyes | q.s. |

**Rezeptur 51: Micellar Water for make-up removal**

| Water | ad 100,0% |
|---|---|
| Perfume | 0,1% |
| Zusammensetzung aus Beispiel 4, 8, 15 oder 16 | 2,0% |
| Capryl/Capramidopropyl Betaine (TEGO® Betain 810, Evonik, 38%) | 1,3% |
| Polyglyceryl-6 Caprylate; Polyglyceryl-3 Cocoate; Polyglyceryl-4 Caprate; Polyglyceryl-6 Ricinoleate (TEGO® Solve 61, Evonik) | 1,0% |
| Betaine (TEGO® Natural Betaine, Evonik) | 2,0% |
| Glycerin (Glycerol EP, vegetable, Spiga Nord) | 1,0% |
| Preservative | q.s. |

**Rezeptur 52: Lösung für wet wipes**

| | |
|---|---|
| Zusammensetzung aus Beispiel 4, 8, 15 oder 16 | 3,5% |
| Aloe Barbadensis Leaf Extract (Aloe-Con UP 40, Florida Food Products Inc.) | 0,2% |
| Isopropyl Myristate (TEGOSOFT® M, Evonik) | 0,2% |
| Disodium Cocoamphodiacetate (REWOTERIC® AM 2 C NM, Evonik, 39%) | 1,5% |
| Perfume | 0,2% |
| Propylene Glycol (Euxyl K 320, Schülke & Mayr GmbH) | 2,5% |
| Hydrolyzed Silk (Crosilk 10000, Croda Inc.) | 0,2% |
| Caprylyl/Capryl Glucoside (Plantacare 810 UP, BASF) | 1,0% |
| Water | ad 100,0% |
| Citric Acid | ad pH 5,0 |
| Phenoxyethanol (S&M Phenoxyethanol, Schülke & Mayr GmbH) | 0,5% |
| Dehydroacetic Acid (Unisept DHA (Universal Preserv-A-Chem, Inc.) | 0,1% |
| Sodium Benzoate (Euxyl K 712, Schülke & Mayr GmbH) | 0,4% |

**Rezeptur 53: Anti-Transpirant Deo**

| | | |
|---|---|---|
| Phase A | Zusammensetzung aus Beispiel 4, 8, 15 oder 16 | 4,0% |
| | Dicaprylyl Ether (Cetiol OE, BASF) | 0,3% |
| | Geraniol (Nerol 800, International Flavors & Fragrances Inc.) | 0,1% |
| | Linalool (Lipofresh, Lipo Chemicals, Inc.) | 0,1% |
| | Perfume | 0,1% |
| Phase B | Propylene Glycol (Euxyl K 320, Schülke & Mayr GmbH) | 1,0% |
| | Butylene Glycol (Oxea Corparation) | 0,2% |
| | Water | 5,0% |
| | Palmitamidopropyltrimonium Chloride (VARISOFT® PATC, Evonik) | 1,0% |
| Phase C | Water | 50,0% |
| | Hydroxethyl Ethylcellulose (Structure Cel 4400 E, AkzoNobel) | 0,8% |
| | Sodium Hydroxide (10% in water) | 0,3% |
| Phase D | Aluminium Chlorohydrate (Locron L, Clariant) | 15,0% |
| Phase E | Preservative | q.s. |
| | Water | ad 100,0% |

**Rezeptur 54: Mundwasser**

| | |
|---|---|
| Zusammensetzung aus Beispiel 4, 8, 15 oder 16 | 0,4% |
| Glycolipids (Rheance One, Evonik) | 0,2% |
| Flavor | 0,2% |
| Water | ad 100,0% |
| Sorbitol (Karion FP Liquid , Merck) | 3,0% |
| Preservative | q.s. |
| Dyes | q.s. |

**Rezeptur 55: Zahnpasta**

| | | |
|---|---|---|
| A | Sorbitol (Karion FP Liquid , Merck) | 50,0% |
| | Water | ad 100,0% |
| | Sodium Carboxymethylcelluslose (Blanose 7MXF, Ashland) | 1,2% |
| B | Sodium Saccharine (Sigma Aldrich) | 0,1% |
| | Sodium Fluoride (Sigma Aldrich) | 0,1% |
| C | Titanium Dioxide (Caesar & Loretz) | 0,4% |
| | Hydrated Silica (Zeodent® 113, Evonik) | 14,0% |
| | Hydrated Silica (Zeodent® 165, Evonik) | 8,0% |
| D | Flavor oil | 1,0% |
| E | Glyceryl Caprylate (Dermosoft® GMCY, Evonik) | 0,3% |
| | Zusammensetzung aus Beispiel 4, 8, 15 oder 16 | 3,5% |

## Patentansprüche

1. Verfahren zur enzymatischen Herstellung einer Mischungszusammensetzung umfassend mindestens zwei ausgewählt aus Zucker-Estern und/oder Zuckeralkohol-Estern umfassend den Verfahrensschritt
B) Umsetzen einer Mischung enthaltend mindestens zwei ausgewählt aus Zuckern und Zuckeralkoholen
mit mindestens einem Acylgruppendonor, bevorzugt Fettsäure-Acylgruppendonor, insbesondere ausgewählt aus Fettsäureestern und Fettsäuren, besonders bevorzugt Fettsäuren,
in Gegenwart einer Lipase.

2. Verfahren gemäß Anspruch 1, **dadurch gekennzeichnet, dass** die Zucker und Zuckeralkohole ausgewählt sind aus der Gruppe Agarose, Allitol, Allulose, Altritol, Amylopectin, Amylose, Arabinitol, Arabinose, Cellobiose, Cellulose, Chitin, Cyclodextrine, Desoxyribose, Dextrane, Erythritol, Fructane, Fructose, Fucose, Galactitol, Galactose, Glucitol, Glucose, Glycogen, Hyaluronsäure, Iditol, Inulin, Isomalt, Isomaltulose, Isomelizitose, Lactitol, Lactose, Lactulose, Maltitol, Maltohexose, Maltopentose, Maltose, Maltotetrose, Maltotriose, Maltulose, Mannitol, Mannose, Melizitose, Pektine, Raffinose, Rhamnose, Ribitol, Ribose, Saccharose, Sorbitol, Sorbose, Stachyose, Stärke, Stärke-Hydrolysate, Threitol, Trehalulose, Umbelliferose, Xylitol und Xylose.

3. Verfahren gemäß Anspruch 1 oder 2, **dadurch gekennzeichnet, dass** der Acylgruppendonor ausgewählt ist aus Fettsäure-Acylgruppendonoren, die insbesondere eine Acylgruppe ausgewählt aus der Gruppe der Acylgruppen von Capronsäure, Caprylsäure, Pelargonsäure, Caprinsäure, Undecylensäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Linolensäure, Petrolesinsäure, Elaidinsäure, Arachinsäure, Behensäure, Erucasäure, Gadoleinsäure, Linolensäure, Eicosapentaensäure, Docosahexaensäure und Arachidonsäure bereitstellen.

4. Verfahren gemäß mindestens einem der vorherigen Ansprüche, **dadurch gekennzeichnet, dass** die in Verfahrensschritt B) eingesetzte Mischung enthaltend mindestens zwei ausgewählt aus Zuckern und Zuckeralkoholen Substanzen ausgewählt aus der Gruppe bestehend aus Cholin-, Ammonium- und Phosphonium-Salzen in einer Menge kleiner 2 Gew.-%, bevorzugt weniger als 1 Gew.-%, besonders bevorzugt weniger als 0,1 Gew.-%, insbesondere keine der Substanzen, aufweist, wobei sich die Gewichtsprozente auf alle Zucker und Zuckeralkohole in Verfahrensschritt B) in der Mischung enthaltend mindestens zwei ausgewählt aus Zuckern und Zuckeralkoholen beziehen.

5. Verfahren gemäß mindestens einem der vorherigen Ansprüche, **dadurch gekennzeichnet, dass** in Verfahrensschritt B) das molare Verhältnis von allen Zuckern und Zuckeralkoholen zu in allen Acylgruppendonoren enthaltenen Acylgruppen in einem Bereich von 1,00 zu 0,08 bis 1,00 zu 10,00 bevorzugt von 1,00 zu 0,500 bis 1,00 zu 7,00, besonders bevorzugt von 1,00 zu 1,25 bis 1,00 zu 2,25, alternativ besonders bevorzugt von 1,00 zu 2,00 bis 1,00 zu 4,50, liegt.

6. Verfahren gemäß mindestens einem der Ansprüche 1 bis 5, **dadurch gekennzeichnet, dass** in Verfahrensschritt B) das molare Verhältnis von allen primären Hydroxylgruppen in allen Zuckern und Zuckeralkoholen zu in allen Acylgruppendonoren enthaltenen Acylgruppen in einem Bereich von 1,00 zu 0,10 bis 1,00 zu 3,00, besonders bevorzugt von 1,00 zu 1,25 bis 1,00 zu 2,25, liegt.

7. Verfahren gemäß mindestens einem der vorherigen Ansprüche, **dadurch gekennzeichnet, dass** die Lipase ausgewählt aus der Gruppe umfassend die Lipase aus *Thermomyces lanuginosus (*accessionnumber *O59952)*, die Lipasen A und B (accessionnumber P41365) aus *Candida antarctica* und, die Lipase aus *Mucor miehei (*accessionnumber *P19515),* die Lipase aus *Humicola sp.* (accessionnumber O59952), die Lipase aus *Rhizomucor javanicus (*accessionnumber S32492), die Lipase aus *Rhizopus oryzae* (accessionnumber P61872), die Lipasen aus *Candida rugosa* (accessionnumber P20261, P32946, P32947, P3294 und P32949), die Lipase aus *Rhizopus niveus (*accessionnumber *P61871),* die Lipase aus *Penicillium camemberti (a*ccessionnumber P25234), die Lipasen aus *Aspergillus niger* (ABG73613, ABG73614 und ABG37906) und die Lipase aus *Penicillium cyclopium (*accessionnumber P61869), sowie jeweils deren auf Aminosäureebene mindestens 60 %, homologen.

8. Verfahren gemäß mindestens einem der vorherigen Ansprüche, **dadurch gekennzeichnet, dass** Verfahrensschritt B) bei Reaktionstemperaturen im Bereich zwischen 20 °C und 160 °C, bevorzugt 35 °C und 130°C, insbesondere zwischen 50 °C und 110 °C, durchgeführt wird.

9. Verfahren gemäß mindestens einem der vorherigen Ansprüche, **dadurch gekennzeichnet, dass** Verfahrensschritt B) bei einem Druck von kleiner 1 bar, bevorzugt kleiner 0,5 bar und besonders bevorzugt kleiner 0,05 bar, durchgeführt wird.

10. Verfahren gemäß mindestens einem der vorherigen Ansprüche, **dadurch gekennzeichnet, dass** in Verfahrensschritt B) die Mischung enthaltend mindestens zwei ausgewählt aus Zuckern und Zuckeralkoholen sowie der Acylgruppendonor in Summe mindestens 10 Gew.-%, bevorzugt mindestens 86 Gew.-%, besonders bevorzugt mindestens 90 Gew.-%, des gesamten Reaktionsansatzes ausmachen.

11. Verfahren gemäß mindestens einem der vorherigen Ansprüche, **dadurch gekennzeichnet, dass** in Verfahrensschritt B) entstehende Nebenprodukte, beispielsweise im Falle, dass der eingesetzte Acylgruppendonor eine Säure ist, Wasser, im Falle, dass der eingesetzte Acylgruppendonor ein Ester ist, der korrespondierende Alkohol, entfernt werden.

12. Verfahren gemäß mindestens einem der vorherigen Ansprüche, **dadurch gekennzeichnet, dass** es den Verfahrensschritt
A) räumlich voneinander getrenntes Bereitstellen der mindestens zwei ausgewählt aus Zuckern und Zuckeralkoholen in fester oder wassergelöster Form und Vermengen derselben zu der in Verfahrensschritt B) eingesetzten Mischung enthaltend mindestens zwei ausgewählt aus Zuckern und Zuckeralkoholen
umfasst.

13. Verfahren gemäß Anspruch 12, **dadurch gekennzeichnet, dass** Verfahrensschritt A) eine Reduktion des Wassergehaltes der in Verfahrensschritt B) eingesetzten Mischung enthaltend mindestens zwei ausgewählt aus Zuckern und Zuckeralkoholen auf weniger als 17 Gew.-%, bevorzugt weniger als 14 Gew.-%, besonders bevorzugt weniger als 10 Gew.-%, umfasst, wobei sich die Gewichtsprozente auf die gesamte in Verfahrensschritt B) eingesetzte Mischung enthaltend mindestens zwei ausgewählt aus Zuckern und Zuckeralkoholen beziehen.

14. Mischungszusammensetzung umfassend mindestens zwei ausgewählt aus Zucker-Estern und Zuckeralkohol-Estern erhältlich nach einem Verfahren gemäß mindestens einem der vorherigen Ansprüche.

15. Mischungszusammensetzung enthaltend Zucker-Ester und/oder Zuckeralkohol-Ester, **dadurch gekennzeichnet, dass** der Zucker- und/oder Zuckeralkoholrest des Zucker-Esters und/oder des Zuckeralkohol-Esters ausgewählt ist aus mindestens zwei Zucker- und/oder Zuckeralkoholresten ausgewählt aus der Gruppe der Reste von Allitol, Allulose, Altritol, Arabinitol, Arabinose, Cellobiose, Desoxyribose, Erythritol, Fructose, Fucose, Galactitol, Galactose, Glucitol, Glucose, Iditol, Isomalt, Isomaltulose, Lactitol, Lactose, Lactulose, Maltitol, Maltose, Maltulose, Mannitol, Mannose, Rhamnose, Ribitol, Ribose, Saccharose, Sorbitol, Sorbose, Threitol, Trehalulose, Xylitol und Xylose
und
der Esterrest ausgewählt ist aus mindestens einer Acylgruppe der Gruppe der Säurereste der Fettsäuren.
